# EUROPÄISCHE PATENTANMELDUNG

(11) **EP 1 239 040 A2**
(43) Veröffentlichungstag der Anmeldung: **11.09.2002**
(21) Anmeldenummer: 02002501.1
(22) Anmeldetag: 02.02.2002
(51) Int. Cl.: C12N 15/31, C07K 14/34, C12N 15/77, C12Q 1/68, C12P 13/08, C12N 1/21

(54) **Mutationen im rpoB-Gen L-Lysin produzierender Corynebacterium glutamicum-Stämme und Verfahren zur Herstellung von L-Lysin**

(30) Priorität: 16.02.2001 DE 10107229; 19.12.2001 DE 10162387
(71) Anmelder: Degussa AG, 40474 Düsseldorf (DE)
(72) Erfinder: Möckel, Bettina, Dr., 40597 Düsseldorf (DE); Bathe, Brigitte, Dr., 33154 Salzkotten (DE); Hermann, Thomas, Dr., 33739 Bielefeld (DE); Pfefferle, Walter, Dr., 33790 Halle (DE); Binder, Michael, Dr., 33803 Steinhagen (Westf.) (DE)

(57) **Zusammenfassung**

Die Erfindung betrifft ein isoliertes Polynukleotid, enthaltend eine Polynukleotidsequenz, ausgewählt aus der Gruppe
a) Polynukleotid, das mindestens zu 70% identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c),
und ein Verfahren zur fermentativen Herstellung von L-Aminosäuren unter Verwendung von coryneformen Bakterien, in denen zumindest das rpoB-Gen verstärkt vorliegt, und die Verwendung von Polynukleotiden, die die erfindungsgemäßen Sequenzen enthalten, als Hybridisierungssonden.

SEQUENZPROTOKOLL

<110>  Degussa AG

<120>  Für das rpoB-Gen kodierende Nukleotidsequenzen

<130>  000781 BT

<160>  12

<170>  PatentIn version 3.1

<210>  1
<211>  5099
<212>  DNA
<213>  Corynebacterium glutamicum

<220>
<221>  CDS
<222>  (702)..(4196)
<223>  rpoB-Wildtypgen

<400>  1
acaatgtgac tcgtgatttt tgggtggatc agcgtaccgg tttggttgtc gatctagctg      60

aaaatattga tgatttttac ggcgaccgca gcggccagaa gtacgaacag aaattgcttt     120

tcgacgcctc cctcgacgat gcagctgtct ctaagctggt tgcacaggcc gaaagcatcc     180

ctgatggaga tgtgagcaaa atcgcaaata ccgtaggtat tgtgatcggt gcggtattgg     240

ctctcgtggg cctggccggg tgttttgggg cgtttgggaa gaaacgtcga gaagcttaac     300

ctgctgttca aatagatttt ccctgtttcg aattgcggaa accccgggtt tgtttgctag     360

ggtgcctcgt agaaggggtc aagaagattt ctgggaaacg cgcccgtgcg gttggttgct     420

aatagcacgc ggagcaccag atgaaaaatc tcccctttac tttcgcgcgc gattggtata     480

ctctgagtcg ttgcgttgga attcgtgact ctttttcgtt cctgtagcgc caagaccttg     540

atcaaggtgg tttaaaaaaa ccgatttgac aaggtcattc agtgctatct ggagtcgttc     600

agggggatcg ggttcctcag cagaccaatt gctcaaaaat accagcggtg ttgatctgca     660

cttaatggcc ttgaccagcc aggtgcaatt acccgcgtga g gtg ctg gaa gga ccc     716
                                              Met Leu Glu Gly Pro
                                              1               5

atc ttg gca gtc tcc cgc cag acc aag tca gtc gtc gat att ccc ggt       764
Ile Leu Ala Val Ser Arg Gln Thr Lys Ser Val Val Asp Ile Pro Gly
                10                  15                  20

gca ccg cag cgt tat tct ttc gcg aag gtg tcc gca ccc att gag gtg       812
Ala Pro Gln Arg Tyr Ser Phe Ala Lys Val Ser Ala Pro Ile Glu Val
            25                  30                  35

ccc ggg cta cta gat ctt caa ctg gat tct tac tcc tgg ctg att ggt       860
Pro Gly Leu Leu Asp Leu Gln Leu Asp Ser Tyr Ser Trp Leu Ile Gly
        40                  45                  50

acg cct gag tgg cgt gct cgt cag aag gaa gaa ttc ggc gag gga gcc 908
Thr Pro Glu Trp Arg Ala Arg Gln Lys Glu Glu Phe Gly Glu Gly Ala
    55                  60                  65

cgc gta acc agc ggc ctt gag aac att ctc gag gag ctc tcc cca atc 956
Arg Val Thr Ser Gly Leu Glu Asn Ile Leu Glu Glu Leu Ser Pro Ile
70                  75                  80                  85

cag gat tac tct gga aac atg tcc ctg agc ctt tcg gag cca cgc ttc 1004
Gln Asp Tyr Ser Gly Asn Met Ser Leu Ser Leu Ser Glu Pro Arg Phe
                90                  95                  100

gaa gac gtc aag aac acc att gac gag gcg aaa gaa aag gac atc aac 1052
Glu Asp Val Lys Asn Thr Ile Asp Glu Ala Lys Glu Lys Asp Ile Asn
            105                 110                 115

tac gcg gcg cca ctg tat gtg acc gcg gag ttc gtc aac aac acc acc 1100
Tyr Ala Ala Pro Leu Tyr Val Thr Ala Glu Phe Val Asn Asn Thr Thr
        120                 125                 130

ggt gaa atc aag tct cag act gtc ttc atc ggc gat ttc cca atg atg 1148
Gly Glu Ile Lys Ser Gln Thr Val Phe Ile Gly Asp Phe Pro Met Met
    135                 140                 145

acg gac aag gga acg ttc atc atc aac gga acc gaa cgc gtt gtg gtc 1196
Thr Asp Lys Gly Thr Phe Ile Ile Asn Gly Thr Glu Arg Val Val Val
150                 155                 160                 165

agc cag ctc gtc cgc tcc ccg ggc gtg tac ttt gac cag acc atc gat 1244
Ser Gln Leu Val Arg Ser Pro Gly Val Tyr Phe Asp Gln Thr Ile Asp
                170                 175                 180

aag tca act gag cgt cca ctg cac gcc gtg aag gtt att cct tcc cgt 1292
Lys Ser Thr Glu Arg Pro Leu His Ala Val Lys Val Ile Pro Ser Arg
            185                 190                 195

ggt gct tgg ctt gag ttt gac gtc gat aag cgc gat tcg gtt ggt gtt 1340
Gly Ala Trp Leu Glu Phe Asp Val Asp Lys Arg Asp Ser Val Gly Val
        200                 205                 210

cgt att gac cgc aag cgt cgc cag cca gtc acc gta ctg ctg aag gct 1388
Arg Ile Asp Arg Lys Arg Arg Gln Pro Val Thr Val Leu Leu Lys Ala
    215                 220                 225

ctt ggc tgg acc act gag cag atc acc gag cgt ttc ggt ttc tct gaa 1436
Leu Gly Trp Thr Thr Glu Gln Ile Thr Glu Arg Phe Gly Phe Ser Glu
230                 235                 240                 245

atc atg atg tcc acc ctc gag tcc gat ggt gta gca aac acc gat gag 1484
Ile Met Met Ser Thr Leu Glu Ser Asp Gly Val Ala Asn Thr Asp Glu
                250                 255                 260

gca ttg ctg gag atc tac cgc aag cag cgt cca ggc gag cag cct acc 1532
Ala Leu Leu Glu Ile Tyr Arg Lys Gln Arg Pro Gly Glu Gln Pro Thr
            265                 270                 275

cgc gac ctt gcg cag tcc ctc ctg gac aac agc ttc ttc cgt gca aag 1580
Arg Asp Leu Ala Gln Ser Leu Leu Asp Asn Ser Phe Phe Arg Ala Lys
        280                 285                 290

cgc tac gac ctg gct cgc gtt ggt cgt tac aag atc aac cgc aag ctc 1628
Arg Tyr Asp Leu Ala Arg Val Gly Arg Tyr Lys Ile Asn Arg Lys Leu
    295                 300                 305

ggc ctt ggt ggc gac cac gat ggt ttg atg act ctt act gaa gag gac 1676
Gly Leu Gly Gly Asp His Asp Gly Leu Met Thr Leu Thr Glu Glu Asp
310                 315                 320                 325

atc gca acc acc atc gag tac ctg gtg cgt ctg cac gca ggt gag cgc 1724
Ile Ala Thr Thr Ile Glu Tyr Leu Val Arg Leu His Ala Gly Glu Arg
                330                 335                 340

gtc atg act tct cca aat ggt gaa gag atc cca gtc gag acc gat gac 1772
Val Met Thr Ser Pro Asn Gly Glu Glu Ile Pro Val Glu Thr Asp Asp
            345                 350                 355

atc gac cac ttt ggt aac cgt cgt ctg cgt acc gtt ggc gaa ctg atc 1820
Ile Asp His Phe Gly Asn Arg Arg Leu Arg Thr Val Gly Glu Leu Ile
        360                 365                 370

cag aac cag gtc cgt gtc ggc ctg tcc cgc atg gag cgc gtt gtt cgt 1868
Gln Asn Gln Val Arg Val Gly Leu Ser Arg Met Glu Arg Val Val Arg
    375                 380                 385

gag cgt atg acc acc cag gat gcg gag tcc att act cct act tcc ttg 1916
Glu Arg Met Thr Thr Gln Asp Ala Glu Ser Ile Thr Pro Thr Ser Leu
390                 395                 400                 405

atc aac gtt cgt cct gtc tct gca gct atc cgt gag ttc ttc gga act 1964
Ile Asn Val Arg Pro Val Ser Ala Ala Ile Arg Glu Phe Phe Gly Thr
                410                 415                 420

tcc cag ctg tct cag ttc atg gac cag aac aac tcc ctg tct ggt ttg 2012
Ser Gln Leu Ser Gln Phe Met Asp Gln Asn Asn Ser Leu Ser Gly Leu
            425                 430                 435

act cac aag cgt cgt ctg tcg gct ctg ggc ccg ggt ggt ctg tcc cgt 2060
Thr His Lys Arg Arg Leu Ser Ala Leu Gly Pro Gly Gly Leu Ser Arg
        440                 445                 450

gag cgc gcc ggc atc gag gtt cga gac gtt cac cca tct cac tac ggc 2108
Glu Arg Ala Gly Ile Glu Val Arg Asp Val His Pro Ser His Tyr Gly
    455                 460                 465

cgt atg tgc cca att gag act ccg gaa ggt cca aac att ggc ctg atc 2156
Arg Met Cys Pro Ile Glu Thr Pro Glu Gly Pro Asn Ile Gly Leu Ile
470                 475                 480                 485

ggt tcc ttg gct tcc tat gct cga gtg aac cca ttc ggt ttc att gag 2204
Gly Ser Leu Ala Ser Tyr Ala Arg Val Asn Pro Phe Gly Phe Ile Glu
                490                 495                 500

acc cca tac cgt cgc atc atc gac ggc aag ctg acc gac cag att gac 2252
Thr Pro Tyr Arg Arg Ile Ile Asp Gly Lys Leu Thr Asp Gln Ile Asp
            505                 510                 515

tac ctt acc gct gat gag gaa gac cgc ttc gtt gtt gcg cag gca aac 2300
Tyr Leu Thr Ala Asp Glu Glu Asp Arg Phe Val Val Ala Gln Ala Asn
        520                 525                 530

acg cac tac gac gaa gag ggc aac atc acc gat gag acc gtc act gtt     2348
Thr His Tyr Asp Glu Glu Gly Asn Ile Thr Asp Glu Thr Val Thr Val
    535                 540                 545

cgt ctg aag gac ggc gac atc gcc atg gtt ggc cgc aac gcg gtt gat     2396
Arg Leu Lys Asp Gly Asp Ile Ala Met Val Gly Arg Asn Ala Val Asp
550                 555                 560                 565

tac atg gac gtt tcc cct cgt cag atg gtt tct gtt ggt acc gcg atg     2444
Tyr Met Asp Val Ser Pro Arg Gln Met Val Ser Val Gly Thr Ala Met
                570                 575                 580

att cca ttc ctg gag cac gac gat gct aac cgt gca ctg atg ggc gcg     2492
Ile Pro Phe Leu Glu His Asp Asp Ala Asn Arg Ala Leu Met Gly Ala
            585                 590                 595

aac atg cag aag cag gct gtg cca ctg att cgt gcc gag gct cct ttc     2540
Asn Met Gln Lys Gln Ala Val Pro Leu Ile Arg Ala Glu Ala Pro Phe
        600                 605                 610

gtg ggc acc ggt atg gag cag cgc gca gca tac gac gcc ggc gac ctg     2588
Val Gly Thr Gly Met Glu Gln Arg Ala Ala Tyr Asp Ala Gly Asp Leu
    615                 620                 625

gtt att acc cca gtc gca ggt gtg gtg gaa aac gtt tca gct gac ttc     2636
Val Ile Thr Pro Val Ala Gly Val Val Glu Asn Val Ser Ala Asp Phe
630                 635                 640                 645

atc acc atc atg gct gat gac ggc aag cgc gaa acc tac ctg ctg cgt     2684
Ile Thr Ile Met Ala Asp Asp Gly Lys Arg Glu Thr Tyr Leu Leu Arg
                650                 655                 660

aag ttc cag cgc acc aac cag ggc acc agc tac aac cag aag cct ttg     2732
Lys Phe Gln Arg Thr Asn Gln Gly Thr Ser Tyr Asn Gln Lys Pro Leu
            665                 670                 675

gtt aac ttg ggc gag cgc gtt gaa gct ggc cag gtt att gct gat ggt     2780
Val Asn Leu Gly Glu Arg Val Glu Ala Gly Gln Val Ile Ala Asp Gly
        680                 685                 690

cca ggt acc ttc aat ggt gaa atg tcc ctt ggc cgt aac ctt ctg gtt     2828
Pro Gly Thr Phe Asn Gly Glu Met Ser Leu Gly Arg Asn Leu Leu Val
    695                 700                 705

gcg ttc atg cct tgg gaa ggc cac aac tac gag gat gcg atc atc ctc     2876
Ala Phe Met Pro Trp Glu Gly His Asn Tyr Glu Asp Ala Ile Ile Leu
710                 715                 720                 725

aac cag aac atc gtt gag cag gac atc ttg acc tcg atc cac atc gag     2924
Asn Gln Asn Ile Val Glu Gln Asp Ile Leu Thr Ser Ile His Ile Glu
                730                 735                 740

gag cac gag atc gat gcc cgc gac act aag ctt ggc gcc gaa gaa atc     2972
Glu His Glu Ile Asp Ala Arg Asp Thr Lys Leu Gly Ala Glu Glu Ile
            745                 750                 755

acc cgc gac atc cct aat gtg tct gaa gaa gtc ctc aag gac ctc gac     3020
Thr Arg Asp Ile Pro Asn Val Ser Glu Glu Val Leu Lys Asp Leu Asp
        760                 765                 770

gac cgc ggt att gtc cgc atc ggt gct gat gtt cgt gac ggc gac atc 3068
Asp Arg Gly Ile Val Arg Ile Gly Ala Asp Val Arg Asp Gly Asp Ile
    775                 780                 785

ctg gtc ggt aag gtc acc cct aag ggc gag acc gag ctc acc ccg gaa 3116
Leu Val Gly Lys Val Thr Pro Lys Gly Glu Thr Glu Leu Thr Pro Glu
790                 795                 800                 805

gag cgc ttg ctg cgc gca atc ttc ggt gag aag gcc cgc gaa gtt cgc 3164
Glu Arg Leu Leu Arg Ala Ile Phe Gly Glu Lys Ala Arg Glu Val Arg
                810                 815                 820

gat acc tcc atg aag gtg cct cac ggt gag acc ggc aag gtc atc ggc 3212
Asp Thr Ser Met Lys Val Pro His Gly Glu Thr Gly Lys Val Ile Gly
            825                 830                 835

gtg cgt cac ttc tcc cgc gag gac gac gac gat ctg gct cct ggc gtc 3260
Val Arg His Phe Ser Arg Glu Asp Asp Asp Asp Leu Ala Pro Gly Val
        840                 845                 850

aac gag atg atc cgt atc tac gtt gct cag aag cgt aag atc cag gac 3308
Asn Glu Met Ile Arg Ile Tyr Val Ala Gln Lys Arg Lys Ile Gln Asp
    855                 860                 865

ggc gat aag ctc gct ggc cgc cac ggt aac aag ggt gtt gtc ggt aaa 3356
Gly Asp Lys Leu Ala Gly Arg His Gly Asn Lys Gly Val Val Gly Lys
870                 875                 880                 885

att ttg cct cag gaa gat atg cca ttc ctt cca gac ggc act cct gtt 3404
Ile Leu Pro Gln Glu Asp Met Pro Phe Leu Pro Asp Gly Thr Pro Val
                890                 895                 900

gac atc atc ttg aac acc cac ggt gtt cca cgt cgt atg aac att ggt 3452
Asp Ile Ile Leu Asn Thr His Gly Val Pro Arg Arg Met Asn Ile Gly
            905                 910                 915

cag gtt ctt gag acc cac ctt ggc tgg ctg gca tct gct ggt tgg tcc 3500
Gln Val Leu Glu Thr His Leu Gly Trp Leu Ala Ser Ala Gly Trp Ser
        920                 925                 930

gtg gat cct gaa gat cct gag aac gct gag ctc gtc aag act ctg cct 3548
Val Asp Pro Glu Asp Pro Glu Asn Ala Glu Leu Val Lys Thr Leu Pro
    935                 940                 945

gca gac ctc ctc gag gtt cct gct ggt tcc ttg act gca act cct gtg 3596
Ala Asp Leu Leu Glu Val Pro Ala Gly Ser Leu Thr Ala Thr Pro Val
950                 955                 960                 965

ttc gac ggt gcg tca aac gaa gag ctc gca ggc ctg ctc gct aat tca 3644
Phe Asp Gly Ala Ser Asn Glu Glu Leu Ala Gly Leu Leu Ala Asn Ser
                970                 975                 980

cgt cca aac cgc gac ggc gac gtc atg gtt aac gcg gat ggt aaa gca 3692
Arg Pro Asn Arg Asp Gly Asp Val Met Val Asn Ala Asp Gly Lys Ala
            985                 990                 995

acg ctt atc gac ggt cgc tcc ggt gag cct tac ccg tac ccg gtt 3737
Thr Leu Ile Asp Gly Arg Ser Gly Glu Pro Tyr Pro Tyr Pro Val
        1000                1005                1010

tcc atc ggc tac atg tac atg ctg aag ctg cac cac ctc gtt gac 3782
Ser Ile Gly Tyr Met Tyr Met Leu Lys Leu His His Leu Val Asp
        1015                1020                1025

gag aag atc cac gca cgt tcc act ggt cct tac tcc atg att acc 3827
Glu Lys Ile His Ala Arg Ser Thr Gly Pro Tyr Ser Met Ile Thr
        1030                1035                1040

cag cag cca ctg ggt ggt aaa gca cag ttc ggt gga cag cgt ttc 3872
Gln Gln Pro Leu Gly Gly Lys Ala Gln Phe Gly Gly Gln Arg Phe
        1045                1050                1055

ggc gaa atg gag gtg tgg gca atg cag gca tac ggc gct gcc tac 3917
Gly Glu Met Glu Val Trp Ala Met Gln Ala Tyr Gly Ala Ala Tyr
        1060                1065                1070

aca ctt cag gag ctg ctg acc atc aag tct gat gac gtg gtt ggc 3962
Thr Leu Gln Glu Leu Leu Thr Ile Lys Ser Asp Asp Val Val Gly
        1075                1080                1085

cgt gtc aag gtc tac gaa gca att gtg aag ggc gag aac atc ccg 4007
Arg Val Lys Val Tyr Glu Ala Ile Val Lys Gly Glu Asn Ile Pro
        1090                1095                1100

gat cca ggt att cct gag tcc ttc aag gtt ctc ctc aag gag ctc 4052
Asp Pro Gly Ile Pro Glu Ser Phe Lys Val Leu Leu Lys Glu Leu
        1105                1110                1115

cag tcc ttg tgc ctg aac gtg gag gtt ctc tcc gca gac ggc act 4097
Gln Ser Leu Cys Leu Asn Val Glu Val Leu Ser Ala Asp Gly Thr
        1120                1125                1130

cca atg gag ctc gcg ggt gac gac gac gac ttc gat cag gca ggc 4142
Pro Met Glu Leu Ala Gly Asp Asp Asp Asp Phe Asp Gln Ala Gly
        1135                1140                1145

gcc tca ctt ggc atc aac ctg tcc cgt gac gag cgt tcc gac gcc 4187
Ala Ser Leu Gly Ile Asn Leu Ser Arg Asp Glu Arg Ser Asp Ala
        1150                1155                1160

gac acc gca tagcagatca gaaaacaacc gctagaaatc aagccataca 4236
Asp Thr Ala
        1165

tcccccggac attgaagaga tgttctgggg ggaaagggag ttttacgtgc tcgacgtaaa 4296

cgtcttcgat gagctccgca tcggcctggc caccgccgac gacatccgcc gttggtccaa 4356

gggtgaggtc aagaagccgg agaccatcaa ctaccgaacc ctcaagcctg agaaggacgg 4416

tctgttctgc gagcgtatct tcggtccaac tcgcgactgg gagtgcgcct gcggtaagta 4476

caagcgtgtc cgctacaagg gcatcatctg tgaacgctgt ggcgttgagg tcaccaagtc 4536

caaggtgcgc cgtgagcgca tgggacacat tgagctcgct gcaccagtaa cccacatttg 4596

gtacttcaag ggcgttccat cacgcctcgg ctaccttttg gaccttgctc caaaggacct 4656

ggacctcatc atctacttcg gtgcgaacat catcaccagc gtggacgaag aggctcgcca 4716

cagcgaccag accactcttg aggcagaaat gcttctggag aagaaggacg ttgaggcaga 4776

cgcagagtct gacattgctg agcgtgctga aaagctcgaa gaggatcttg ctgaacttga 4836

ggcagctggc gctaaggccg acgctcgccg caaggttcag gctgctgccg ataaggaaat 4896

gcagcacatc cgtgagcgtg cacagcgcga aatcgatcgt ctcgatgagg tctggcagac 4956

cttcatcaag cttgctccaa agcagatgat ccgcgatgag aagctctacg atgaactgat 5016

cgaccgctac gaggattact tcaccggtgg tatgggtgca gagtccattg aggctttgat 5076

ccagaacttc gaccttgatg ctg 5099

<210> 2
<211> 1165
<212> PRT
<213> Corynebacterium glutamicum

<400> 2
Met Leu Glu Gly Pro Ile Leu Ala Val Ser Arg Gln Thr Lys Ser Val
1               5                   10                  15

Val Asp Ile Pro Gly Ala Pro Gln Arg Tyr Ser Phe Ala Lys Val Ser
            20                  25                  30

Ala Pro Ile Glu Val Pro Gly Leu Leu Asp Leu Gln Leu Asp Ser Tyr
        35                  40                  45

Ser Trp Leu Ile Gly Thr Pro Glu Trp Arg Ala Arg Gln Lys Glu Glu
    50                  55                  60

Phe Gly Glu Gly Ala Arg Val Thr Ser Gly Leu Glu Asn Ile Leu Glu
65                  70                  75                  80

Glu Leu Ser Pro Ile Gln Asp Tyr Ser Gly Asn Met Ser Leu Ser Leu
                85                  90                  95

Ser Glu Pro Arg Phe Glu Asp Val Lys Asn Thr Ile Asp Glu Ala Lys
            100                 105                 110

Glu Lys Asp Ile Asn Tyr Ala Ala Pro Leu Tyr Val Thr Ala Glu Phe
        115                 120                 125

Val Asn Asn Thr Thr Gly Glu Ile Lys Ser Gln Thr Val Phe Ile Gly
    130                 135                 140

Asp Phe Pro Met Met Thr Asp Lys Gly Thr Phe Ile Ile Asn Gly Thr
145                 150                 155                 160

Glu Arg Val Val Val Ser Gln Leu Val Arg Ser Pro Gly Val Tyr Phe
                165                 170                 175

Asp Gln Thr Ile Asp Lys Ser Thr Glu Arg Pro Leu His Ala Val Lys
            180                 185                 190

Val Ile Pro Ser Arg Gly Ala Trp Leu Glu Phe Asp Val Asp Lys Arg
        195                 200                 205

Asp Ser Val Gly Val Arg Ile Asp Arg Lys Arg Arg Gln Pro Val Thr
    210                 215                 220

Val Leu Leu Lys Ala Leu Gly Trp Thr Thr Glu Gln Ile Thr Glu Arg
225                 230                 235                 240

Phe Gly Phe Ser Glu Ile Met Met Ser Thr Leu Glu Ser Asp Gly Val
                245                 250                 255

Ala Asn Thr Asp Glu Ala Leu Leu Glu Ile Tyr Arg Lys Gln Arg Pro
            260                 265                 270

Gly Glu Gln Pro Thr Arg Asp Leu Ala Gln Ser Leu Leu Asp Asn Ser
        275                 280                 285

Phe Phe Arg Ala Lys Arg Tyr Asp Leu Ala Arg Val Gly Arg Tyr Lys
    290                 295                 300

Ile Asn Arg Lys Leu Gly Leu Gly Gly Asp His Asp Gly Leu Met Thr
305                 310                 315                 320

Leu Thr Glu Glu Asp Ile Ala Thr Thr Ile Glu Tyr Leu Val Arg Leu
                325                 330                 335

His Ala Gly Glu Arg Val Met Thr Ser Pro Asn Gly Glu Glu Ile Pro
            340                 345                 350

Val Glu Thr Asp Asp Ile Asp His Phe Gly Asn Arg Arg Leu Arg Thr
        355                 360                 365

Val Gly Glu Leu Ile Gln Asn Gln Val Arg Val Gly Leu Ser Arg Met
    370                 375                 380

Glu Arg Val Val Arg Glu Arg Met Thr Thr Gln Asp Ala Glu Ser Ile
385                 390                 395                 400

Thr Pro Thr Ser Leu Ile Asn Val Arg Pro Val Ser Ala Ala Ile Arg
                405                 410                 415

Glu Phe Phe Gly Thr Ser Gln Leu Ser Gln Phe Met Asp Gln Asn Asn
            420                 425                 430

Ser Leu Ser Gly Leu Thr His Lys Arg Arg Leu Ser Ala Leu Gly Pro
        435                 440                 445

Gly Gly Leu Ser Arg Glu Arg Ala Gly Ile Glu Val Arg Asp Val His
    450                 455                 460

Pro Ser His Tyr Gly Arg Met Cys Pro Ile Glu Thr Pro Glu Gly Pro
465                 470                 475                 480

Asn Ile Gly Leu Ile Gly Ser Leu Ala Ser Tyr Ala Arg Val Asn Pro
                485                 490                 495

Phe Gly Phe Ile Glu Thr Pro Tyr Arg Arg Ile Ile Asp Gly Lys Leu
            500                 505                 510

Thr Asp Gln Ile Asp Tyr Leu Thr Ala Asp Glu Glu Asp Arg Phe Val
        515                 520                 525

Val Ala Gln Ala Asn Thr His Tyr Asp Glu Glu Gly Asn Ile Thr Asp
    530                 535                 540

Glu Thr Val Thr Val Arg Leu Lys Asp Gly Asp Ile Ala Met Val Gly
545                 550                 555                 560

Arg Asn Ala Val Asp Tyr Met Asp Val Ser Pro Arg Gln Met Val Ser
                565                 570                 575

Val Gly Thr Ala Met Ile Pro Phe Leu Glu His Asp Asp Ala Asn Arg
            580                 585                 590

Ala Leu Met Gly Ala Asn Met Gln Lys Gln Ala Val Pro Leu Ile Arg
        595                 600                 605

Ala Glu Ala Pro Phe Val Gly Thr Gly Met Glu Gln Arg Ala Ala Tyr
    610                 615                 620

Asp Ala Gly Asp Leu Val Ile Thr Pro Val Ala Gly Val Val Glu Asn
625                 630                 635                 640

Val Ser Ala Asp Phe Ile Thr Ile Met Ala Asp Asp Gly Lys Arg Glu
                645                 650                 655

Thr Tyr Leu Leu Arg Lys Phe Gln Arg Thr Asn Gln Gly Thr Ser Tyr
            660                 665                 670

Asn Gln Lys Pro Leu Val Asn Leu Gly Glu Arg Val Glu Ala Gly Gln
        675                 680                 685

Val Ile Ala Asp Gly Pro Gly Thr Phe Asn Gly Glu Met Ser Leu Gly
    690                 695                 700

Arg Asn Leu Leu Val Ala Phe Met Pro Trp Glu Gly His Asn Tyr Glu
705                 710                 715                 720

Asp Ala Ile Ile Leu Asn Gln Asn Ile Val Glu Gln Asp Ile Leu Thr
                725                 730                 735

Ser Ile His Ile Glu Glu His Glu Ile Asp Ala Arg Asp Thr Lys Leu
            740                 745                 750

Gly Ala Glu Glu Ile Thr Arg Asp Ile Pro Asn Val Ser Glu Glu Val
        755                 760                 765

Leu Lys Asp Leu Asp Asp Arg Gly Ile Val Arg Ile Gly Ala Asp Val
    770                 775                 780

Arg Asp Gly Asp Ile Leu Val Gly Lys Val Thr Pro Lys Gly Glu Thr
785                 790                 795                 800

Glu Leu Thr Pro Glu Glu Arg Leu Leu Arg Ala Ile Phe Gly Glu Lys
                805                 810                 815

Ala Arg Glu Val Arg Asp Thr Ser Met Lys Val Pro His Gly Glu Thr
            820                 825                 830

Gly Lys Val Ile Gly Val Arg His Phe Ser Arg Glu Asp Asp Asp Asp
        835                 840                 845

Leu Ala Pro Gly Val Asn Glu Met Ile Arg Ile Tyr Val Ala Gln Lys
    850                 855                 860

Arg Lys Ile Gln Asp Gly Asp Lys Leu Ala Gly Arg His Gly Asn Lys
865                 870                 875                 880

Gly Val Val Gly Lys Ile Leu Pro Gln Glu Asp Met Pro Phe Leu Pro
                885                 890                 895

Asp Gly Thr Pro Val Asp Ile Ile Leu Asn Thr His Gly Val Pro Arg
            900                 905                 910

Arg Met Asn Ile Gly Gln Val Leu Glu Thr His Leu Gly Trp Leu Ala
        915                 920                 925

Ser Ala Gly Trp Ser Val Asp Pro Glu Asp Pro Glu Asn Ala Glu Leu
    930                 935                 940

Val Lys Thr Leu Pro Ala Asp Leu Leu Glu Val Pro Ala Gly Ser Leu
945                 950                 955                 960

Thr Ala Thr Pro Val Phe Asp Gly Ala Ser Asn Glu Glu Leu Ala Gly
                965                 970                 975

Leu Leu Ala Asn Ser Arg Pro Asn Arg Asp Gly Asp Val Met Val Asn
            980                 985                 990

Ala Asp Gly Lys Ala Thr Leu Ile  Asp Gly Arg Ser Gly  Glu Pro Tyr
        995                 1000                1005

Pro Tyr  Pro Val Ser Ile Gly  Tyr Met Tyr Met Leu  Lys Leu His
    1010                1015                1020

His Leu  Val Asp Glu Lys Ile  His Ala Arg Ser Thr  Gly Pro Tyr
    1025                1030                1035

Ser Met  Ile Thr Gln Gln Pro  Leu Gly Gly Lys Ala  Gln Phe Gly
    1040                1045                1050

Gly Gln  Arg Phe Gly Glu Met  Glu Val Trp Ala Met  Gln Ala Tyr
    1055                1060                1065

Gly Ala  Ala Tyr Thr Leu Gln  Glu Leu Leu Thr Ile  Lys Ser Asp
    1070                1075                1080

Asp Val  Val Gly Arg Val Lys  Val Tyr Glu Ala Ile  Val Lys Gly
    1085                1090                1095

Glu Asn  Ile Pro Asp Pro Gly  Ile Pro Glu Ser Phe  Lys Val Leu
    1100                1105                1110

Leu Lys  Glu Leu Gln Ser Leu  Cys Leu Asn Val Glu  Val Leu Ser
    1115                1120                1125

Ala Asp  Gly Thr Pro Met Glu  Leu Ala Gly Asp Asp  Asp Asp Phe
    1130                1135                1140

Asp Gln  Ala Gly Ala Ser Leu  Gly Ile Asn Leu Ser  Arg Asp Glu
    1145                1150                1155

Arg Ser  Asp Ala Asp Thr Ala
    1160                1165

<210>  3
<211>  5099
<212>  DNA
<213>  Corynebacterium glutamicum

<220>
<221> CDS
<222> (702)..(4196)

<223> rpoB-Allel 1547

<220>
<221> mutation
<222> (1987)..(1987)
<223> Austausch von Adenin gegen Thymin

<220>
<221> mutation
<222> (1288)..(1288)
<223> Austausch von Cytosin gegen Thymin

<220>
<221> mutation
<222> (715)..(715)
<223> Austausch von Cytosin gegen Thymin

<400> 3
   acaatgtgac tcgtgatttt tgggtggatc agcgtaccgg tttggttgtc gatctagctg 60
   aaaatattga tgatttttac ggcgaccgca gcggccagaa gtacgaacag aaattgcttt 120
   tcgacgcctc cctcgacgat gcagctgtct ctaagctggt tgcacaggcc gaaagcatcc 180
   ctgatggaga tgtgagcaaa atcgcaaata ccgtaggtat tgtgatcggt gcggtattgg 240
   ctctcgtggg cctggccggg tgttttgggg cgtttgggaa gaaacgtcga gaagcttaac 300
   ctgctgttca aatagatttt ccctgtttcg aattgcggaa accccgggtt tgtttgctag 360
   ggtgcctcgt agaaggggtc aagaagattt ctgggaaacg cgcccgtgcg gttggttgct 420
   aatagcacgc ggagcaccag atgaaaaatc tcccctttac tttcgcgcgc gattggtata 480
   ctctgagtcg ttgcgttgga attcgtgact ctttttcgtt cctgtagcgc caagaccttg 540
   atcaaggtgg tttaaaaaaa ccgatttgac aaggtcattc agtgctatct ggagtcgttc 600
   agggggatcg ggttcctcag cagaccaatt gctcaaaaat accagcggtg ttgatctgca 660
   cttaatggcc ttgaccagcc aggtgcaatt acccgcgtga g gtg ctg gaa gga ctc 716
   Met Leu Glu Gly Leu
   1 5
   atc ttg gca gtc tcc cgc cag acc aag tca gtc gtc gat att ccc ggt 764
   Ile Leu Ala Val Ser Arg Gln Thr Lys Ser Val Val Asp Ile Pro Gly
   10 15 20
   gca ccg cag cgt tat tct ttc gcg aag gtg tcc gca ccc att gag gtg 812
   Ala Pro Gln Arg Tyr Ser Phe Ala Lys Val Ser Ala Pro Ile Glu Val
   25 30 35
   ccc ggg cta cta gat ctt caa ctg gat tct tac tcc tgg ctg att ggt 860
   Pro Gly Leu Leu Asp Leu Gln Leu Asp Ser Tyr Ser Trp Leu Ile Gly
   40 45 50
   acg cct gag tgg cgt gct cgt cag aag gaa gaa ttc ggc gag gga gcc 908
   Thr Pro Glu Trp Arg Ala Arg Gln Lys Glu Glu Phe Gly Glu Gly Ala
   55 60 65
   cgc gta acc agc ggc ctt gag aac att ctc gag gag ctc tcc cca atc 956
   Arg Val Thr Ser Gly Leu Glu Asn Ile Leu Glu Glu Leu Ser Pro Ile
   70 75 80 85
   cag gat tac tct gga aac atg tcc ctg agc ctt tcg gag cca cgc ttc 1004
   Gln Asp Tyr Ser Gly Asn Met Ser Leu Ser Leu Ser Glu Pro Arg Phe
   90 95 100
   gaa gac gtc aag aac acc att gac gag gcg aaa gaa aag gac atc aac 1052
   Glu Asp Val Lys Asn Thr Ile Asp Glu Ala Lys Glu Lys Asp Ile Asn
   105 110 115
   tac gcg gcg cca ctg tat gtg acc gcg gag ttc gtc aac aac acc acc 1100
   Tyr Ala Ala Pro Leu Tyr Val Thr Ala Glu Phe Val Asn Asn Thr Thr
   120 125 130
   ggt gaa atc aag tct cag act gtc ttc atc ggc gat ttc cca atg atg 1148
   Gly Glu Ile Lys Ser Gln Thr Val Phe Ile Gly Asp Phe Pro Met Met
   135 140 145
   acg gac aag gga acg ttc atc atc aac gga acc gaa cgc gtt gtg gtc 1196
   Thr Asp Lys Gly Thr Phe Ile Ile Asn Gly Thr Glu Arg Val Val Val
   150 155 160 165
   agc cag ctc gtc cgc tcc ccg ggc gtg tac ttt gac cag acc atc gat 1244
   Ser Gln Leu Val Arg Ser Pro Gly Val Tyr Phe Asp Gln Thr Ile Asp
   170 175 180
   aag tca act gag cgt cca ctg cac gcc gtg aag gtt att cct ttc cgt 1292
   Lys Ser Thr Glu Arg Pro Leu His Ala Val Lys Val Ile Pro Phe Arg
   185 190 195
   ggt gct tgg ctt gag ttt gac gtc gat aag cgc gat tcg gtt ggt gtt 1340
   Gly Ala Trp Leu Glu Phe Asp Val Asp Lys Arg Asp Ser Val Gly Val
   200 205 210
   cgt att gac cgc aag cgt cgc cag cca gtc acc gta ctg ctg aag gct 1388
   Arg Ile Asp Arg Lys Arg Arg Gln Pro Val Thr Val Leu Leu Lys Ala
   215 220 225
   ctt ggc tgg acc act gag cag atc acc gag cgt ttc ggt ttc tct gaa 1436
   Leu Gly Trp Thr Thr Glu Gln Ile Thr Glu Arg Phe Gly Phe Ser Glu
   230 235 240 245
   atc atg atg tcc acc ctc gag tcc gat ggt gta gca aac acc gat gag 1484
   Ile Met Met Ser Thr Leu Glu Ser Asp Gly Val Ala Asn Thr Asp Glu
   250 255 260
   gca ttg ctg gag atc tac cgc aag cag cgt cca ggc gag cag cct acc 1532
   Ala Leu Leu Glu Ile Tyr Arg Lys Gln Arg Pro Gly Glu Gln Pro Thr
   265 270 275
   cgc gac ctt gcg cag tcc ctc ctg gac aac agc ttc ttc cgt gca aag 1580
   Arg Asp Leu Ala Gln Ser Leu Leu Asp Asn Ser Phe Phe Arg Ala Lys
   280 285 290
   cgc tac gac ctg gct cgc gtt ggt cgt tac aag atc aac cgc aag ctc 1628
   Arg Tyr Asp Leu Ala Arg Val Gly Arg Tyr Lys Ile Asn Arg Lys Leu
   295 300 305
   ggc ctt ggt ggc gac cac gat ggt ttg atg act ctt act gaa gag gac 1676
   Gly Leu Gly Gly Asp His Asp Gly Leu Met Thr Leu Thr Glu Glu Asp
   310 315 320 325
   atc gca acc acc atc gag tac ctg gtg cgt ctg cac gca ggt gag cgc 1724
   Ile Ala Thr Thr Ile Glu Tyr Leu Val Arg Leu His Ala Gly Glu Arg
   330 335 340
   gtc atg act tct cca aat ggt gaa gag atc cca gtc gag acc gat gac 1772
   Val Met Thr Ser Pro Asn Gly Glu Glu Ile Pro Val Glu Thr Asp Asp
   345 350 355
   atc gac cac ttt ggt aac cgt cgt ctg cgt acc gtt ggc gaa ctg atc 1820
   Ile Asp His Phe Gly Asn Arg Arg Leu Arg Thr Val Gly Glu Leu Ile
   360 365 370
   cag aac cag gtc cgt gtc ggc ctg tcc cgc atg gag cgc gtt gtt cgt 1868
   Gln Asn Gln Val Arg Val Gly Leu Ser Arg Met Glu Arg Val Val Arg
   375 380 385
   gag cgt atg acc acc cag gat gcg gag tcc att act cct act tcc ttg 1916
   Glu Arg Met Thr Thr Gln Asp Ala Glu Ser Ile Thr Pro Thr Ser Leu
   390 395 400 405
   atc aac gtt cgt cct gtc tct gca gct atc cgt gag ttc ttc gga act 1964
   Ile Asn Val Arg Pro Val Ser Ala Ala Ile Arg Glu Phe Phe Gly Thr
   410 415 420
   tcc cag ctg tct cag ttc atg gtc cag aac aac tcc ctg tct ggt ttg 2012
   Ser Gln Leu Ser Gln Phe Met Val Gln Asn Asn Ser Leu Ser Gly Leu
   425 430 435
   act cac aag cgt cgt ctg tcg gct ctg ggc ccg ggt ggt ctg tcc cgt 2060
   Thr His Lys Arg Arg Leu Ser Ala Leu Gly Pro Gly Gly Leu Ser Arg
   440 445 450
   gag cgc gcc ggc atc gag gtt cga gac gtt cac cca tct cac tac ggc 2108
   Glu Arg Ala Gly Ile Glu Val Arg Asp Val His Pro Ser His Tyr Gly
   455 460 465
   cgt atg tgc cca att gag act ccg gaa ggt cca aac att ggc ctg atc 2156
   Arg Met Cys Pro Ile Glu Thr Pro Glu Gly Pro Asn Ile Gly Leu Ile
   470 475 480 485
   ggt tcc ttg gct tcc tat gct cga gtg aac cca ttc ggt ttc att gag 2204
   Gly Ser Leu Ala Ser Tyr Ala Arg Val Asn Pro Phe Gly Phe Ile Glu
   490 495 500
   acc cca tac cgt cgc atc atc gac ggc aag ctg acc gac cag att gac 2252
   Thr Pro Tyr Arg Arg Ile Ile Asp Gly Lys Leu Thr Asp Gln Ile Asp
   505 510 515
   tac ctt acc gct gat gag gaa gac cgc ttc gtt gtt gcg cag gca aac 2300
   Tyr Leu Thr Ala Asp Glu Glu Asp Arg Phe Val Val Ala Gln Ala Asn
   520 525 530
   acg cac tac gac gaa gag ggc aac atc acc gat gag acc gtc act gtt 2348
   Thr His Tyr Asp Glu Glu Gly Asn Ile Thr Asp Glu Thr Val Thr Val
   535 540 545
   cgt ctg aag gac ggc gac atc gcc atg gtt ggc cgc aac gcg gtt gat 2396
   Arg Leu Lys Asp Gly Asp Ile Ala Met Val Gly Arg Asn Ala Val Asp
   550 555 560 565
   tac atg gac gtt tcc cct cgt cag atg gtt tct gtt ggt acc gcg atg 2444
   Tyr Met Asp Val Ser Pro Arg Gln Met Val Ser Val Gly Thr Ala Met
   570 575 580
   att cca ttc ctg gag cac gac gat gct aac cgt gca ctg atg ggc gcg 2492
   Ile Pro Phe Leu Glu His Asp Asp Ala Asn Arg Ala Leu Met Gly Ala
   585 590 595
   aac atg cag aag cag gct gtg cca ctg att cgt gcc gag gct cct ttc 2540
   Asn Met Gln Lys Gln Ala Val Pro Leu Ile Arg Ala Glu Ala Pro Phe
   600 605 610
   gtg ggc acc ggt atg gag cag cgc gca gca tac gac gcc ggc gac ctg 2588
   Val Gly Thr Gly Met Glu Gln Arg Ala Ala Tyr Asp Ala Gly Asp Leu
   615 620 625
   gtt att acc cca gtc gca ggt gtg gtg gaa aac gtt tca gct gac ttc 2636
   Val Ile Thr Pro Val Ala Gly Val Val Glu Asn Val Ser Ala Asp Phe
   630 635 640 645
   atc acc atc atg gct gat gac ggc aag cgc gaa acc tac ctg ctg cgt 2684
   Ile Thr Ile Met Ala Asp Asp Gly Lys Arg Glu Thr Tyr Leu Leu Arg
   650 655 660
   aag ttc cag cgc acc aac cag ggc acc agc tac aac cag aag cct ttg 2732
   Lys Phe Gln Arg Thr Asn Gln Gly Thr Ser Tyr Asn Gln Lys Pro Leu
   665 670 675
   gtt aac ttg ggc gag cgc gtt gaa gct ggc cag gtt att gct gat ggt 2780
   Val Asn Leu Gly Glu Arg Val Glu Ala Gly Gln Val Ile Ala Asp Gly
   680 685 690
   cca ggt acc ttc aat ggt gaa atg tcc ctt ggc cgt aac ctt ctg gtt 2828
   Pro Gly Thr Phe Asn Gly Glu Met Ser Leu Gly Arg Asn Leu Leu Val
   695 700 705
   gcg ttc atg cct tgg gaa ggc cac aac tac gag gat gcg atc atc ctc 2876
   Ala Phe Met Pro Trp Glu Gly His Asn Tyr Glu Asp Ala Ile Ile Leu
   710 715 720 725
   aac cag aac atc gtt gag cag gac atc ttg acc tcg atc cac atc gag 2924
   Asn Gln Asn Ile Val Glu Gln Asp Ile Leu Thr Ser Ile His Ile Glu
   730 735 740
   gag cac gag atc gat gcc cgc gac act aag ctt ggc gcc gaa gaa atc 2972
   Glu His Glu Ile Asp Ala Arg Asp Thr Lys Leu Gly Ala Glu Glu Ile
   745 750 755
   acc cgc gac atc cct aat gtg tct gaa gaa gtc ctc aag gac ctc gac 3020
   Thr Arg Asp Ile Pro Asn Val Ser Glu Glu Val Leu Lys Asp Leu Asp
   760 765 770
   gac cgc ggt att gtc cgc atc ggt gct gat gtt cgt gac ggc gac atc 3068
   Asp Arg Gly Ile Val Arg Ile Gly Ala Asp Val Arg Asp Gly Asp Ile
   775 780 785
   ctg gtc ggt aag gtc acc cct aag ggc gag acc gag ctc acc ccg gaa 3116
   Leu Val Gly Lys Val Thr Pro Lys Gly Glu Thr Glu Leu Thr Pro Glu
   790 795 800 805
   gag cgc ttg ctg cgc gca atc ttc ggt gag aag gcc cgc gaa gtt cgc 3164
   Glu Arg Leu Leu Arg Ala Ile Phe Gly Glu Lys Ala Arg Glu Val Arg
   810 815 820
   gat acc tcc atg aag gtg cct cac ggt gag acc ggc aag gtc atc ggc 3212
   Asp Thr Ser Met Lys Val Pro His Gly Glu Thr Gly Lys Val Ile Gly
   825 830 835
   gtg cgt cac ttc tcc cgc gag gac gac gac gat ctg gct cct ggc gtc 3260
   Val Arg His Phe Ser Arg Glu Asp Asp Asp Asp Leu Ala Pro Gly Val
   840 845 850
   aac gag atg atc cgt atc tac gtt gct cag aag cgt aag atc cag gac 3308
   Asn Glu Met Ile Arg Ile Tyr Val Ala Gln Lys Arg Lys Ile Gln Asp
   855 860 865
   ggc gat aag ctc gct ggc cgc cac ggt aac aag ggt gtt gtc ggt aaa 3356
   Gly Asp Lys Leu Ala Gly Arg His Gly Asn Lys Gly Val Val Gly Lys
   870 875 880 885
   att ttg cct cag gaa gat atg cca ttc ctt cca gac ggc act cct gtt 3404
   Ile Leu Pro Gln Glu Asp Met Pro Phe Leu Pro Asp Gly Thr Pro Val
   890 895 900
   gac atc atc ttg aac acc cac ggt gtt cca cgt cgt atg aac att ggt 3452
   Asp Ile Ile Leu Asn Thr His Gly Val Pro Arg Arg Met Asn Ile Gly
   905 910 915
   cag gtt ctt gag acc cac ctt ggc tgg ctg gca tct gct ggt tgg tcc 3500
   Gln Val Leu Glu Thr His Leu Gly Trp Leu Ala Ser Ala Gly Trp Ser
   920 925 930
   gtg gat cct gaa gat cct gag aac gct gag ctc gtc aag act ctg cct 3548
   Val Asp Pro Glu Asp Pro Glu Asn Ala Glu Leu Val Lys Thr Leu Pro
   935 940 945
   gca gac ctc ctc gag gtt cct gct ggt tcc ttg act gca act cct gtg 3596
   Ala Asp Leu Leu Glu Val Pro Ala Gly Ser Leu Thr Ala Thr Pro Val
   950 955 960 965
   ttc gac ggt gcg tca aac gaa gag ctc gca ggc ctg ctc gct aat tca 3644
   Phe Asp Gly Ala Ser Asn Glu Glu Leu Ala Gly Leu Leu Ala Asn Ser
   970 975 980
   cgt cca aac cgc gac ggc gac gtc atg gtt aac gcg gat ggt aaa gca 3692
   Arg Pro Asn Arg Asp Gly Asp Val Met Val Asn Ala Asp Gly Lys Ala
   985 990 995
   acg ctt atc gac ggt cgc tcc ggt gag cct tac ccg tac ccg gtt 3737
   Thr Leu Ile Asp Gly Arg Ser Gly Glu Pro Tyr Pro Tyr Pro Val
   1000 1005 1010
   tcc atc ggc tac atg tac atg ctg aag ctg cac cac ctc gtt gac 3782
   Ser Ile Gly Tyr Met Tyr Met Leu Lys Leu His His Leu Val Asp
   1015 1020 1025
   gag aag atc cac gca cgt tcc act ggt cct tac tcc atg att acc 3827
   Glu Lys Ile His Ala Arg Ser Thr Gly Pro Tyr Ser Met Ile Thr
   1030 1035 1040
   cag cag cca ctg ggt ggt aaa gca cag ttc ggt gga cag cgt ttc 3872
   Gln Gln Pro Leu Gly Gly Lys Ala Gln Phe Gly Gly Gln Arg Phe
   1045 1050 1055
   ggc gaa atg gag gtg tgg gca atg cag gca tac ggc gct gcc tac 3917
   Gly Glu Met Glu Val Trp Ala Met Gln Ala Tyr Gly Ala Ala Tyr
   1060 1065 1070
   aca ctt cag gag ctg ctg acc atc aag tct gat gac gtg gtt ggc 3962
   Thr Leu Gln Glu Leu Leu Thr Ile Lys Ser Asp Asp Val Val Gly
   1075 1080 1085
   cgt gtc aag gtc tac gaa gca att gtg aag ggc gag aac atc ccg 4007
   Arg Val Lys Val Tyr Glu Ala Ile Val Lys Gly Glu Asn Ile Pro
   1090 1095 1100
   gat cca ggt att cct gag tcc ttc aag gtt ctc ctc aag gag ctc 4052
   Asp Pro Gly Ile Pro Glu Ser Phe Lys Val Leu Leu Lys Glu Leu
   1105 1110 1115
   cag tcc ttg tgc ctg aac gtg gag gtt ctc tcc gca gac ggc act 4097
   Gln Ser Leu Cys Leu Asn Val Glu Val Leu Ser Ala Asp Gly Thr
   1120 1125 1130
   cca atg gag ctc gcg ggt gac gac gac gac ttc gat cag gca ggc 4142
   Pro Met Glu Leu Ala Gly Asp Asp Asp Asp Phe Asp Gln Ala Gly
   1135 1140 1145
   gcc tca ctt ggc atc aac ctg tcc cgt gac gag cgt tcc gac gcc 4187
   Ala Ser Leu Gly Ile Asn Leu Ser Arg Asp Glu Arg Ser Asp Ala
   1150 1155 1160
   gac acc gca tagcagatca gaaaacaacc gctagaaatc aagccataca 4236
   Asp Thr Ala
   1165
   tcccccggac attgaagaga tgttctgggg ggaaagggag ttttacgtgc tcgacgtaaa 4296
   cgtcttcgat gagctccgca tcggcctggc caccgccgac gacatccgcc gttggtccaa 4356
   gggtgaggtc aagaagccgg agaccatcaa ctaccgaacc ctcaagcctg agaaggacgg 4416
   tctgttctgc gagcgtatct tcggtccaac tcgcgactgg gagtgcgcct gcggtaagta 4476
   caagcgtgtc cgctacaagg gcatcatctg tgaacgctgt ggcgttgagg tcaccaagtc 4536
   caaggtgcgc cgtgagcgca tgggacacat tgagctcgct gcaccagtaa cccacatttg 4596
   gtacttcaag ggcgttccat cacgcctcgg ctaccttttg gaccttgctc caaaggacct 4656
   ggacctcatc atctacttcg gtgcgaacat catcaccagc gtggacgaag aggctcgcca 4716
   cagcgaccag accactcttg aggcagaaat gcttctggag aagaaggacg ttgaggcaga 4776
   cgcagagtct gacattgctg agcgtgctga aaagctcgaa gaggatcttg ctgaacttga 4836
   ggcagctggc gctaaggccg acgctcgccg caaggttcag gctgctgccg ataaggaaat 4896
   gcagcacatc cgtgagcgtg cacagcgcga aatcgatcgt ctcgatgagg tctggcagac 4956
   cttcatcaag cttgctccaa agcagatgat ccgcgatgag aagctctacg atgaactgat 5016
   cgaccgctac gaggattact tcaccggtgg tatgggtgca gagtccattg aggctttgat 5076
   ccagaacttc gaccttgatg ctg 5099

<210> 4
<211> 1165
<212> PRT
<213> Corynebacterium glutamicum

<400> 4
Met Leu Glu Gly Leu Ile Leu Ala Val Ser Arg Gln Thr Lys Ser Val
1               5                   10                  15

Val Asp Ile Pro Gly Ala Pro Gln Arg Tyr Ser Phe Ala Lys Val Ser
            20                  25                  30

Ala Pro Ile Glu Val Pro Gly Leu Leu Asp Leu Gln Leu Asp Ser Tyr
        35                  40                  45

Ser Trp Leu Ile Gly Thr Pro Glu Trp Arg Ala Arg Gln Lys Glu Glu
    50                  55                  60

Phe Gly Glu Gly Ala Arg Val Thr Ser Gly Leu Glu Asn Ile Leu Glu
65                  70                  75                  80

Glu Leu Ser Pro Ile Gln Asp Tyr Ser Gly Asn Met Ser Leu Ser Leu
                85                  90                  95

Ser Glu Pro Arg Phe Glu Asp Val Lys Asn Thr Ile Asp Glu Ala Lys
            100                 105                 110

Glu Lys Asp Ile Asn Tyr Ala Ala Pro Leu Tyr Val Thr Ala Glu Phe
        115                 120                 125

Val Asn Asn Thr Thr Gly Glu Ile Lys Ser Gln Thr Val Phe Ile Gly
    130                 135                 140

Asp Phe Pro Met Met Thr Asp Lys Gly Thr Phe Ile Ile Asn Gly Thr
145                 150                 155                 160

Glu Arg Val Val Val Ser Gln Leu Val Arg Ser Pro Gly Val Tyr Phe
                165                 170                 175

Asp Gln Thr Ile Asp Lys Ser Thr Glu Arg Pro Leu His Ala Val Lys
            180                 185                 190

Val Ile Pro Phe Arg Gly Ala Trp Leu Glu Phe Asp Val Asp Lys Arg
        195                 200                 205

Asp Ser Val Gly Val Arg Ile Asp Arg Lys Arg Arg Gln Pro Val Thr
    210                 215                 220

Val Leu Leu Lys Ala Leu Gly Trp Thr Thr Glu Gln Ile Thr Glu Arg
225                 230                 235                 240

Phe Gly Phe Ser Glu Ile Met Met Ser Thr Leu Glu Ser Asp Gly Val
                245                 250                 255

Ala Asn Thr Asp Glu Ala Leu Leu Glu Ile Tyr Arg Lys Gln Arg Pro
            260                 265                 270

Gly Glu Gln Pro Thr Arg Asp Leu Ala Gln Ser Leu Leu Asp Asn Ser
        275                 280                 285

Phe Phe Arg Ala Lys Arg Tyr Asp Leu Ala Arg Val Gly Arg Tyr Lys
    290                 295                 300

Ile Asn Arg Lys Leu Gly Leu Gly Gly Asp His Asp Gly Leu Met Thr
305                 310                 315                 320

Leu Thr Glu Glu Asp Ile Ala Thr Thr Ile Glu Tyr Leu Val Arg Leu
                325                 330                 335

His Ala Gly Glu Arg Val Met Thr Ser Pro Asn Gly Glu Glu Ile Pro
            340                 345                 350

Val Glu Thr Asp Asp Ile Asp His Phe Gly Asn Arg Arg Leu Arg Thr
        355                 360                 365

Val Gly Glu Leu Ile Gln Asn Gln Val Arg Val Gly Leu Ser Arg Met
    370                 375                 380

Glu Arg Val Val Arg Glu Arg Met Thr Thr Gln Asp Ala Glu Ser Ile
385                 390                 395                 400

Thr Pro Thr Ser Leu Ile Asn Val Arg Pro Val Ser Ala Ala Ile Arg
                405                 410                 415

Glu Phe Phe Gly Thr Ser Gln Leu Ser Gln Phe Met Val Gln Asn Asn
            420                 425                 430

Ser Leu Ser Gly Leu Thr His Lys Arg Arg Leu Ser Ala Leu Gly Pro
        435                 440                 445

Gly Gly Leu Ser Arg Glu Arg Ala Gly Ile Glu Val Arg Asp Val His
    450                 455                 460

Pro Ser His Tyr Gly Arg Met Cys Pro Ile Glu Thr Pro Glu Gly Pro
465                 470                 475                 480

Asn Ile Gly Leu Ile Gly Ser Leu Ala Ser Tyr Ala Arg Val Asn Pro
                485                 490                 495

Phe Gly Phe Ile Glu Thr Pro Tyr Arg Arg Ile Ile Asp Gly Lys Leu
            500                 505                 510

Thr Asp Gln Ile Asp Tyr Leu Thr Ala Asp Glu Glu Asp Arg Phe Val
        515                 520                 525

Val Ala Gln Ala Asn Thr His Tyr Asp Glu Glu Gly Asn Ile Thr Asp
    530                 535                 540

Glu Thr Val Thr Val Arg Leu Lys Asp Gly Asp Ile Ala Met Val Gly
545                 550                 555                 560

Arg Asn Ala Val Asp Tyr Met Asp Val Ser Pro Arg Gln Met Val Ser
                565                 570                 575

Val Gly Thr Ala Met Ile Pro Phe Leu Glu His Asp Asp Ala Asn Arg
            580                 585                 590

Ala Leu Met Gly Ala Asn Met Gln Lys Gln Ala Val Pro Leu Ile Arg
        595                 600                 605

Ala Glu Ala Pro Phe Val Gly Thr Gly Met Glu Gln Arg Ala Ala Tyr
    610                 615                 620

Asp Ala Gly Asp Leu Val Ile Thr Pro Val Ala Gly Val Val Glu Asn
625                 630                 635                 640

Val Ser Ala Asp Phe Ile Thr Ile Met Ala Asp Asp Gly Lys Arg Glu
                645                 650                 655

Thr Tyr Leu Leu Arg Lys Phe Gln Arg Thr Asn Gln Gly Thr Ser Tyr
            660                 665                 670

Asn Gln Lys Pro Leu Val Asn Leu Gly Glu Arg Val Glu Ala Gly Gln
        675                 680                 685

Val Ile Ala Asp Gly Pro Gly Thr Phe Asn Gly Glu Met Ser Leu Gly
    690                 695                 700

Arg Asn Leu Leu Val Ala Phe Met Pro Trp Glu Gly His Asn Tyr Glu
705                 710                 715                 720

Asp Ala Ile Ile Leu Asn Gln Asn Ile Val Glu Gln Asp Ile Leu Thr
                725                 730                 735

Ser Ile His Ile Glu Glu His Glu Ile Asp Ala Arg Asp Thr Lys Leu
            740                 745                 750

Gly Ala Glu Glu Ile Thr Arg Asp Ile Pro Asn Val Ser Glu Glu Val
        755                 760                 765

Leu Lys Asp Leu Asp Asp Arg Gly Ile Val Arg Ile Gly Ala Asp Val
    770                 775                 780

Arg Asp Gly Asp Ile Leu Val Gly Lys Val Thr Pro Lys Gly Glu Thr
785                 790                 795                 800

Glu Leu Thr Pro Glu Glu Arg Leu Leu Arg Ala Ile Phe Gly Glu Lys
                805                 810                 815

Ala Arg Glu Val Arg Asp Thr Ser Met Lys Val Pro His Gly Glu Thr
            820                 825                 830

Gly Lys Val Ile Gly Val Arg His Phe Ser Arg Glu Asp Asp Asp Asp
        835                 840                 845

Leu Ala Pro Gly Val Asn Glu Met Ile Arg Ile Tyr Val Ala Gln Lys
    850                 855                 860

Arg Lys Ile Gln Asp Gly Asp Lys Leu Ala Gly Arg His Gly Asn Lys
865                 870                 875                 880

Gly Val Val Gly Lys Ile Leu Pro Gln Glu Asp Met Pro Phe Leu Pro
                885                 890                 895

Asp Gly Thr Pro Val Asp Ile Ile Leu Asn Thr His Gly Val Pro Arg
            900                 905                 910

Arg Met Asn Ile Gly Gln Val Leu Glu Thr His Leu Gly Trp Leu Ala
        915                 920                 925

Ser Ala Gly Trp Ser Val Asp Pro Glu Asp Pro Glu Asn Ala Glu Leu
    930                 935                 940

Val Lys Thr Leu Pro Ala Asp Leu Leu Glu Val Pro Ala Gly Ser Leu
945                 950                 955                 960

Thr Ala Thr Pro Val Phe Asp Gly Ala Ser Asn Glu Glu Leu Ala Gly
                965                 970                 975

Leu Leu Ala Asn Ser Arg Pro Asn Arg Asp Gly Asp Val Met Val Asn
            980                 985                 990

Ala Asp Gly Lys Ala Thr Leu Ile Asp Gly Arg Ser Gly Glu Pro Tyr
        995                 1000                1005

Pro Tyr Pro Val Ser Ile Gly Tyr Met Tyr Met Leu Lys Leu His
    1010                1015                1020

His Leu Val Asp Glu Lys Ile His Ala Arg Ser Thr Gly Pro Tyr
    1025                1030                1035

Ser Met Ile Thr Gln Gln Pro Leu Gly Gly Lys Ala Gln Phe Gly
    1040                1045                1050

Gly Gln Arg Phe Gly Glu Met Glu Val Trp Ala Met Gln Ala Tyr
    1055                1060                1065

Gly Ala Ala Tyr Thr Leu Gln Glu Leu Leu Thr Ile Lys Ser Asp
    1070                1075                1080

Asp Val Val Gly Arg Val Lys Val Tyr Glu Ala Ile Val Lys Gly
    1085                1090                1095

Glu Asn Ile Pro Asp Pro Gly Ile Pro Glu Ser Phe Lys Val Leu
    1100                1105                1110

Leu Lys Glu Leu Gln Ser Leu Cys Leu Asn Val Glu Val Leu Ser
    1115                1120                1125

Ala Asp Gly Thr Pro Met Glu Leu Ala Gly Asp Asp Asp Asp Phe
    1130                1135                1140

Asp Gln Ala Gly Ala Ser Leu Gly Ile Asn Leu Ser Arg Asp Glu
    1145                1150                1155

Arg Ser Asp Ala Asp Thr Ala
    1160                1165

<210> 5
<211> 5099
<212> DNA
<213> Corynebacterium glutamicum

<220>
<221> CDS
<222> (702)..(4196)
<223> rpoB-Allel 1546

<220>
<221> mutation
<222> (2016)..(2016)
<223> Austausch von Cytosin gegen Thymin

<400> 5
acaatgtgac tcgtgatttt tgggtggatc agcgtaccgg tttggttgtc gatctagctg 60

aaaatattga tgatttttac ggcgaccgca gcggccagaa gtacgaacag aaattgcttt 120

tcgacgcctc cctcgacgat gcagctgtct ctaagctggt tgcacaggcc gaaagcatcc 180

ctgatggaga tgtgagcaaa atcgcaaata ccgtaggtat tgtgatcggt gcggtattgg 240

ctctcgtggg cctggccggg tgttttgggg cgtttgggaa gaaacgtcga gaagcttaac 300

ctgctgttca aatagatttt ccctgtttcg aattgcggaa accccgggtt tgtttgctag 360

ggtgcctcgt agaaggggtc aagaagattt ctgggaaacg cgcccgtgcg gttggttgct 420

aatagcacgc ggagcaccag atgaaaaatc tcccctttac tttcgcgcgc gattggtata 480

ctctgagtcg ttgcgttgga attcgtgact ctttttcgtt cctgtagcgc caagaccttg 540

atcaaggtgg tttaaaaaaa ccgatttgac aaggtcattc agtgctatct ggagtcgttc 600

agggggatcg ggttcctcag cagaccaatt gctcaaaaat accagcggtg ttgatctgca 660

cttaatggcc ttgaccagcc aggtgcaatt acccgcgtga g gtg ctg gaa gga ccc 716
                                              Met Leu Glu Gly Pro
                                              1               5

atc ttg gca gtc tcc cgc cag acc aag tca gtc gtc gat att ccc ggt 764
Ile Leu Ala Val Ser Arg Gln Thr Lys Ser Val Val Asp Ile Pro Gly
                10                  15                  20

gca ccg cag cgt tat tct ttc gcg aag gtg tcc gca ccc att gag gtg 812
Ala Pro Gln Arg Tyr Ser Phe Ala Lys Val Ser Ala Pro Ile Glu Val
            25                  30                  35

ccc ggg cta cta gat ctt caa ctg gat tct tac tcc tgg ctg att ggt 860
Pro Gly Leu Leu Asp Leu Gln Leu Asp Ser Tyr Ser Trp Leu Ile Gly
        40                  45                  50

acg cct gag tgg cgt gct cgt cag aag gaa gaa ttc ggc gag gga gcc 908
Thr Pro Glu Trp Arg Ala Arg Gln Lys Glu Glu Phe Gly Glu Gly Ala
    55                  60                  65

cgc gta acc agc ggc ctt gag aac att ctc gag gag ctc tcc cca atc 956
Arg Val Thr Ser Gly Leu Glu Asn Ile Leu Glu Glu Leu Ser Pro Ile
70                  75                  80                  85

cag gat tac tct gga aac atg tcc ctg agc ctt tcg gag cca cgc ttc 1004
Gln Asp Tyr Ser Gly Asn Met Ser Leu Ser Leu Ser Glu Pro Arg Phe
                90                  95                  100

gaa gac gtc aag aac acc att gac gag gcg aaa gaa aag gac atc aac 1052
Glu Asp Val Lys Asn Thr Ile Asp Glu Ala Lys Glu Lys Asp Ile Asn
            105                 110                 115

tac gcg gcg cca ctg tat gtg acc gcg gag ttc gtc aac aac acc acc 1100
Tyr Ala Ala Pro Leu Tyr Val Thr Ala Glu Phe Val Asn Asn Thr Thr
        120                 125                 130

ggt gaa atc aag tct cag act gtc ttc atc ggc gat ttc cca atg atg 1148
Gly Glu Ile Lys Ser Gln Thr Val Phe Ile Gly Asp Phe Pro Met Met
    135                 140                 145

acg gac aag gga acg ttc atc atc aac gga acc gaa cgc gtt gtg gtc 1196
Thr Asp Lys Gly Thr Phe Ile Ile Asn Gly Thr Glu Arg Val Val Val
150                 155                 160                 165

agc cag ctc gtc cgc tcc ccg ggc gtg tac ttt gac cag acc atc gat 1244
Ser Gln Leu Val Arg Ser Pro Gly Val Tyr Phe Asp Gln Thr Ile Asp
                170                 175                 180

aag tca act gag cgt cca ctg cac gcc gtg aag gtt att cct tcc cgt 1292
Lys Ser Thr Glu Arg Pro Leu His Ala Val Lys Val Ile Pro Ser Arg
            185                 190                 195

ggt gct tgg ctt gag ttt gac gtc gat aag cgc gat tcg gtt ggt gtt 1340
Gly Ala Trp Leu Glu Phe Asp Val Asp Lys Arg Asp Ser Val Gly Val
        200                 205                 210

cgt att gac cgc aag cgt cgc cag cca gtc acc gta ctg ctg aag gct 1388
Arg Ile Asp Arg Lys Arg Arg Gln Pro Val Thr Val Leu Leu Lys Ala
    215                 220                 225

ctt ggc tgg acc act gag cag atc acc gag cgt ttc ggt ttc tct gaa 1436
Leu Gly Trp Thr Thr Glu Gln Ile Thr Glu Arg Phe Gly Phe Ser Glu
230                 235                 240                 245

atc atg atg tcc acc ctc gag tcc gat ggt gta gca aac acc gat gag 1484
Ile Met Met Ser Thr Leu Glu Ser Asp Gly Val Ala Asn Thr Asp Glu
                250                 255                 260

gca ttg ctg gag atc tac cgc aag cag cgt cca ggc gag cag cct acc 1532
Ala Leu Leu Glu Ile Tyr Arg Lys Gln Arg Pro Gly Glu Gln Pro Thr
            265                 270                 275

cgc gac ctt gcg cag tcc ctc ctg gac aac agc ttc ttc cgt gca aag 1580
Arg Asp Leu Ala Gln Ser Leu Leu Asp Asn Ser Phe Phe Arg Ala Lys
        280                 285                 290

cgc tac gac ctg gct cgc gtt ggt cgt tac aag atc aac cgc aag ctc 1628
Arg Tyr Asp Leu Ala Arg Val Gly Arg Tyr Lys Ile Asn Arg Lys Leu
    295                 300                 305

ggc ctt ggt ggc gac cac gat ggt ttg atg act ctt act gaa gag gac 1676
Gly Leu Gly Gly Asp His Asp Gly Leu Met Thr Leu Thr Glu Glu Asp
310                 315                 320                 325

atc gca acc acc atc gag tac ctg gtg cgt ctg cac gca ggt gag cgc 1724
Ile Ala Thr Thr Ile Glu Tyr Leu Val Arg Leu His Ala Gly Glu Arg
                330                 335                 340

gtc atg act tct cca aat ggt gaa gag atc cca gtc gag acc gat gac 1772
Val Met Thr Ser Pro Asn Gly Glu Glu Ile Pro Val Glu Thr Asp Asp
            345                 350                 355

atc gac cac ttt ggt aac cgt cgt ctg cgt acc gtt ggc gaa ctg atc 1820
Ile Asp His Phe Gly Asn Arg Arg Leu Arg Thr Val Gly Glu Leu Ile
        360                 365                 370

cag aac cag gtc cgt gtc ggc ctg tcc cgc atg gag cgc gtt gtt cgt 1868
Gln Asn Gln Val Arg Val Gly Leu Ser Arg Met Glu Arg Val Val Arg
    375                 380                 385

gag cgt atg acc acc cag gat gcg gag tcc att act cct act tcc ttg 1916
Glu Arg Met Thr Thr Gln Asp Ala Glu Ser Ile Thr Pro Thr Ser Leu
390                 395                 400                 405

atc aac gtt cgt cct gtc tct gca gct atc cgt gag ttc ttc gga act 1964
Ile Asn Val Arg Pro Val Ser Ala Ala Ile Arg Glu Phe Phe Gly Thr
                410                 415                 420

tcc cag ctg tct cag ttc atg gac cag aac aac tcc ctg tct ggt ttg 2012
Ser Gln Leu Ser Gln Phe Met Asp Gln Asn Asn Ser Leu Ser Gly Leu
            425                 430                 435

act tac aag cgt cgt ctg tcg gct ctg ggc ccg ggt ggt ctg tcc cgt 2060
Thr Tyr Lys Arg Arg Leu Ser Ala Leu Gly Pro Gly Gly Leu Ser Arg
        440                 445                 450

gag cgc gcc ggc atc gag gtt cga gac gtt cac cca tct cac tac ggc 2108
Glu Arg Ala Gly Ile Glu Val Arg Asp Val His Pro Ser His Tyr Gly
    455                 460                 465

cgt atg tgc cca att gag act ccg gaa ggt cca aac att ggc ctg atc 2156
Arg Met Cys Pro Ile Glu Thr Pro Glu Gly Pro Asn Ile Gly Leu Ile
470                 475                 480                 485

ggt tcc ttg gct tcc tat gct cga gtg aac cca ttc ggt ttc att gag 2204
Gly Ser Leu Ala Ser Tyr Ala Arg Val Asn Pro Phe Gly Phe Ile Glu
                490                 495                 500

acc cca tac cgt cgc atc atc gac ggc aag ctg acc gac cag att gac 2252
Thr Pro Tyr Arg Arg Ile Ile Asp Gly Lys Leu Thr Asp Gln Ile Asp
            505                 510                 515

tac ctt acc gct gat gag gaa gac cgc ttc gtt gtt gcg cag gca aac 2300
Tyr Leu Thr Ala Asp Glu Glu Asp Arg Phe Val Val Ala Gln Ala Asn
        520                 525                 530

acg cac tac gac gaa gag ggc aac atc acc gat gag acc gtc act gtt 2348
Thr His Tyr Asp Glu Glu Gly Asn Ile Thr Asp Glu Thr Val Thr Val
    535                 540                 545

cgt ctg aag gac ggc gac atc gcc atg gtt ggc cgc aac gcg gtt gat 2396
Arg Leu Lys Asp Gly Asp Ile Ala Met Val Gly Arg Asn Ala Val Asp
550                 555                 560                 565

tac atg gac gtt tcc cct cgt cag atg gtt tct gtt ggt acc gcg atg 2444
Tyr Met Asp Val Ser Pro Arg Gln Met Val Ser Val Gly Thr Ala Met
                570                 575                 580

att cca ttc ctg gag cac gac gat gct aac cgt gca ctg atg ggc gcg 2492
Ile Pro Phe Leu Glu His Asp Asp Ala Asn Arg Ala Leu Met Gly Ala
            585                 590                 595

aac atg cag aag cag gct gtg cca ctg att cgt gcc gag gct cct ttc 2540
Asn Met Gln Lys Gln Ala Val Pro Leu Ile Arg Ala Glu Ala Pro Phe
        600                 605                 610

gtg ggc acc ggt atg gag cag cgc gca gca tac gac gcc ggc gac ctg 2588
Val Gly Thr Gly Met Glu Gln Arg Ala Ala Tyr Asp Ala Gly Asp Leu
    615                 620                 625

gtt att acc cca gtc gca ggt gtg gtg gaa aac gtt tca gct gac ttc 2636
Val Ile Thr Pro Val Ala Gly Val Val Glu Asn Val Ser Ala Asp Phe
630                 635                 640                 645

atc acc atc atg gct gat gac ggc aag cgc gaa acc tac ctg ctg cgt 2684
Ile Thr Ile Met Ala Asp Asp Gly Lys Arg Glu Thr Tyr Leu Leu Arg
                650                 655                 660

aag ttc cag cgc acc aac cag ggc acc agc tac aac cag aag cct ttg 2732
Lys Phe Gln Arg Thr Asn Gln Gly Thr Ser Tyr Asn Gln Lys Pro Leu
            665                 670                 675

gtt aac ttg ggc gag cgc gtt gaa gct ggc cag gtt att gct gat ggt 2780
Val Asn Leu Gly Glu Arg Val Glu Ala Gly Gln Val Ile Ala Asp Gly
        680                 685                 690

cca ggt acc ttc aat ggt gaa atg tcc ctt ggc cgt aac ctt ctg gtt 2828
Pro Gly Thr Phe Asn Gly Glu Met Ser Leu Gly Arg Asn Leu Leu Val
    695                 700                 705

gcg ttc atg cct tgg gaa ggc cac aac tac gag gat gcg atc atc ctc 2876
Ala Phe Met Pro Trp Glu Gly His Asn Tyr Glu Asp Ala Ile Ile Leu
710                 715                 720                 725

aac cag aac atc gtt gag cag gac atc ttg acc tcg atc cac atc gag 2924
Asn Gln Asn Ile Val Glu Gln Asp Ile Leu Thr Ser Ile His Ile Glu
                730                 735                 740

gag cac gag atc gat gcc cgc gac act aag ctt ggc gcc gaa gaa atc 2972
Glu His Glu Ile Asp Ala Arg Asp Thr Lys Leu Gly Ala Glu Glu Ile
            745                 750                 755

acc cgc gac atc cct aat gtg tct gaa gaa gtc ctc aag gac ctc gac 3020
Thr Arg Asp Ile Pro Asn Val Ser Glu Glu Val Leu Lys Asp Leu Asp
        760                 765                 770

gac cgc ggt att gtc cgc atc ggt gct gat gtt cgt gac ggc gac atc 3068
Asp Arg Gly Ile Val Arg Ile Gly Ala Asp Val Arg Asp Gly Asp Ile
    775                 780                 785

ctg gtc ggt aag gtc acc cct aag ggc gag acc gag ctc acc ccg gaa 3116
Leu Val Gly Lys Val Thr Pro Lys Gly Glu Thr Glu Leu Thr Pro Glu
790                 795                 800                 805

gag cgc ttg ctg cgc gca atc ttc ggt gag aag gcc cgc gaa gtt cgc 3164
Glu Arg Leu Leu Arg Ala Ile Phe Gly Glu Lys Ala Arg Glu Val Arg
                810                 815                 820

gat acc tcc atg aag gtg cct cac ggt gag acc ggc aag gtc atc ggc 3212
Asp Thr Ser Met Lys Val Pro His Gly Glu Thr Gly Lys Val Ile Gly
            825                 830                 835

gtg cgt cac ttc tcc cgc gag gac gac gac gat ctg gct cct ggc gtc 3260
Val Arg His Phe Ser Arg Glu Asp Asp Asp Asp Leu Ala Pro Gly Val
        840                 845                 850

aac gag atg atc cgt atc tac gtt gct cag aag cgt aag atc cag gac 3308
Asn Glu Met Ile Arg Ile Tyr Val Ala Gln Lys Arg Lys Ile Gln Asp
    855                 860                 865

ggc gat aag ctc gct ggc cgc cac ggt aac aag ggt gtt gtc ggt aaa 3356
Gly Asp Lys Leu Ala Gly Arg His Gly Asn Lys Gly Val Val Gly Lys
870                 875                 880                 885

att ttg cct cag gaa gat atg cca ttc ctt cca gac ggc act cct gtt 3404
Ile Leu Pro Gln Glu Asp Met Pro Phe Leu Pro Asp Gly Thr Pro Val
                890                 895                 900

gac atc atc ttg aac acc cac ggt gtt cca cgt cgt atg aac att ggt 3452
Asp Ile Ile Leu Asn Thr His Gly Val Pro Arg Arg Met Asn Ile Gly
            905                 910                 915

cag gtt ctt gag acc cac ctt ggc tgg ctg gca tct gct ggt tgg tcc 3500
Gln Val Leu Glu Thr His Leu Gly Trp Leu Ala Ser Ala Gly Trp Ser
        920                 925                 930

gtg gat cct gaa gat cct gag aac gct gag ctc gtc aag act ctg cct 3548
Val Asp Pro Glu Asp Pro Glu Asn Ala Glu Leu Val Lys Thr Leu Pro
    935                 940                 945

gca gac ctc ctc gag gtt cct gct ggt tcc ttg act gca act cct gtg 3596
Ala Asp Leu Leu Glu Val Pro Ala Gly Ser Leu Thr Ala Thr Pro Val
950                 955                 960                 965

ttc gac ggt gcg tca aac gaa gag ctc gca ggc ctg ctc gct aat tca 3644
Phe Asp Gly Ala Ser Asn Glu Glu Leu Ala Gly Leu Leu Ala Asn Ser
                970                 975                 980

cgt cca aac cgc gac ggc gac gtc atg gtt aac gcg gat ggt aaa gca 3692
Arg Pro Asn Arg Asp Gly Asp Val Met Val Asn Ala Asp Gly Lys Ala
            985                 990                 995

acg ctt atc gac ggt cgc tcc ggt gag cct tac ccg tac ccg gtt 3737
Thr Leu Ile Asp Gly Arg Ser Gly Glu Pro Tyr Pro Tyr Pro Val
        1000                1005                1010

tcc atc ggc tac atg tac atg ctg aag ctg cac cac ctc gtt gac 3782
Ser Ile Gly Tyr Met Tyr Met Leu Lys Leu His His Leu Val Asp
        1015                1020                1025

gag aag atc cac gca cgt tcc act ggt cct tac tcc atg att acc 3827
Glu Lys Ile His Ala Arg Ser Thr Gly Pro Tyr Ser Met Ile Thr
        1030                1035                1040

cag cag cca ctg ggt ggt aaa gca cag ttc ggt gga cag cgt ttc 3872
Gln Gln Pro Leu Gly Gly Lys Ala Gln Phe Gly Gly Gln Arg Phe
        1045                1050                1055

ggc gaa atg gag gtg tgg gca atg cag gca tac ggc gct gcc tac 3917
Gly Glu Met Glu Val Trp Ala Met Gln Ala Tyr Gly Ala Ala Tyr
        1060                1065                1070

aca ctt cag gag ctg ctg acc atc aag tct gat gac gtg gtt ggc 3962
Thr Leu Gln Glu Leu Leu Thr Ile Lys Ser Asp Asp Val Val Gly
        1075                1080                1085

cgt gtc aag gtc tac gaa gca att gtg aag ggc gag aac atc ccg 4007
Arg Val Lys Val Tyr Glu Ala Ile Val Lys Gly Glu Asn Ile Pro
        1090                1095                1100

gat cca ggt att cct gag tcc ttc aag gtt ctc ctc aag gag ctc 4052
Asp Pro Gly Ile Pro Glu Ser Phe Lys Val Leu Leu Lys Glu Leu
        1105                1110                1115

cag tcc ttg tgc ctg aac gtg gag gtt ctc tcc gca gac ggc act 4097
Gln Ser Leu Cys Leu Asn Val Glu Val Leu Ser Ala Asp Gly Thr
        1120                1125                1130

cca atg gag ctc gcg ggt gac gac gac gac ttc gat cag gca ggc 4142
Pro Met Glu Leu Ala Gly Asp Asp Asp Asp Phe Asp Gln Ala Gly
        1135                1140                1145

gcc tca ctt ggc atc aac ctg tcc cgt gac gag cgt tcc gac gcc 4187
Ala Ser Leu Gly Ile Asn Leu Ser Arg Asp Glu Arg Ser Asp Ala
        1150                1155                1160

gac acc gca tagcagatca gaaaacaacc gctagaaatc aagccataca 4236
Asp Thr Ala
        1165

tcccccggac attgaagaga tgttctgggg ggaaagggag ttttacgtgc tcgacgtaaa 4296

cgtcttcgat gagctccgca tcggcctggc caccgccgac gacatccgcc gttggtccaa 4356

gggtgaggtc aagaagccgg agaccatcaa ctaccgaacc ctcaagcctg agaaggacgg 4416

tctgttctgc gagcgtatct tcggtccaac tcgcgactgg gagtgcgcct gcggtaagta 4476

caagcgtgtc cgctacaagg gcatcatctg tgaacgctgt ggcgttgagg tcaccaagtc 4536

caaggtgcgc cgtgagcgca tgggacacat tgagctcgct gcaccagtaa cccacatttg 4596

gtacttcaag ggcgttccat cacgcctcgg ctaccttttg gaccttgctc caaaggacct 4656

ggacctcatc atctacttcg gtgcgaacat catcaccagc gtggacgaag aggctcgcca 4716

cagcgaccag accactcttg aggcagaaat gcttctggag aagaaggacg ttgaggcaga 4776

cgcagagtct gacattgctg agcgtgctga aaagctcgaa gaggatcttg ctgaacttga 4836

ggcagctggc gctaaggccg acgctcgccg caaggttcag gctgctgccg ataaggaaat 4896

gcagcacatc cgtgagcgtg cacagcgcga aatcgatcgt ctcgatgagg tctggcagac 4956

cttcatcaag cttgctccaa agcagatgat ccgcgatgag aagctctacg atgaactgat 5016

cgaccgctac gaggattact tcaccggtgg tatgggtgca gagtccattg aggctttgat 5076

ccagaacttc gaccttgatg ctg 5099

<210> 6
<211> 1165
<212> PRT

<213>  Corynebacterium glutamicum

<400>  6
Met Leu Glu Gly Pro Ile Leu Ala Val Ser Arg Gln Thr Lys Ser Val
1               5                   10                  15

Val Asp Ile Pro Gly Ala Pro Gln Arg Tyr Ser Phe Ala Lys Val Ser
            20                  25                  30

Ala Pro Ile Glu Val Pro Gly Leu Leu Asp Leu Gln Leu Asp Ser Tyr
        35                  40                  45

Ser Trp Leu Ile Gly Thr Pro Glu Trp Arg Ala Arg Gln Lys Glu Glu
    50                  55                  60

Phe Gly Glu Gly Ala Arg Val Thr Ser Gly Leu Glu Asn Ile Leu Glu
65                  70                  75                  80

Glu Leu Ser Pro Ile Gln Asp Tyr Ser Gly Asn Met Ser Leu Ser Leu
                85                  90                  95

Ser Glu Pro Arg Phe Glu Asp Val Lys Asn Thr Ile Asp Glu Ala Lys
            100                 105                 110

Glu Lys Asp Ile Asn Tyr Ala Ala Pro Leu Tyr Val Thr Ala Glu Phe
        115                 120                 125

Val Asn Asn Thr Thr Gly Glu Ile Lys Ser Gln Thr Val Phe Ile Gly
    130                 135                 140

Asp Phe Pro Met Met Thr Asp Lys Gly Thr Phe Ile Ile Asn Gly Thr
145                 150                 155                 160

Glu Arg Val Val Val Ser Gln Leu Val Arg Ser Pro Gly Val Tyr Phe
                165                 170                 175

Asp Gln Thr Ile Asp Lys Ser Thr Glu Arg Pro Leu His Ala Val Lys
            180                 185                 190

Val Ile Pro Ser Arg Gly Ala Trp Leu Glu Phe Asp Val Asp Lys Arg
        195                 200                 205

Asp Ser Val Gly Val Arg Ile Asp Arg Lys Arg Arg Gln Pro Val Thr
    210                 215                 220

Val Leu Leu Lys Ala Leu Gly Trp Thr Thr Glu Gln Ile Thr Glu Arg
225                 230                 235                 240

Phe Gly Phe Ser Glu Ile Met Met Ser Thr Leu Glu Ser Asp Gly Val
                245                 250                 255

Ala Asn Thr Asp Glu Ala Leu Leu Glu Ile Tyr Arg Lys Gln Arg Pro
            260                 265                 270

Gly Glu Gln Pro Thr Arg Asp Leu Ala Gln Ser Leu Leu Asp Asn Ser
        275                 280                 285

Phe Phe Arg Ala Lys Arg Tyr Asp Leu Ala Arg Val Gly Arg Tyr Lys
    290                 295                 300

Ile Asn Arg Lys Leu Gly Leu Gly Gly Asp His Asp Gly Leu Met Thr
305                 310                 315                 320

Leu Thr Glu Glu Asp Ile Ala Thr Thr Ile Glu Tyr Leu Val Arg Leu
                325                 330                 335

His Ala Gly Glu Arg Val Met Thr Ser Pro Asn Gly Glu Glu Ile Pro
            340                 345                 350

Val Glu Thr Asp Asp Ile Asp His Phe Gly Asn Arg Arg Leu Arg Thr
        355                 360                 365

Val Gly Glu Leu Ile Gln Asn Gln Val Arg Val Gly Leu Ser Arg Met
    370                 375                 380

Glu Arg Val Val Arg Glu Arg Met Thr Thr Gln Asp Ala Glu Ser Ile
385                 390                 395                 400

Thr Pro Thr Ser Leu Ile Asn Val Arg Pro Val Ser Ala Ala Ile Arg
                405                 410                 415

Glu Phe Phe Gly Thr Ser Gln Leu Ser Gln Phe Met Asp Gln Asn Asn
            420                 425                 430

Ser Leu Ser Gly Leu Thr Tyr Lys Arg Arg Leu Ser Ala Leu Gly Pro
        435                 440                 445

Gly Gly Leu Ser Arg Glu Arg Ala Gly Ile Glu Val Arg Asp Val His
    450                 455                 460

Pro Ser His Tyr Gly Arg Met Cys Pro Ile Glu Thr Pro Glu Gly Pro
465                 470                 475                 480

Asn Ile Gly Leu Ile Gly Ser Leu Ala Ser Tyr Ala Arg Val Asn Pro
                485                 490                 495

Phe Gly Phe Ile Glu Thr Pro Tyr Arg Arg Ile Ile Asp Gly Lys Leu
            500                 505                 510

Thr Asp Gln Ile Asp Tyr Leu Thr Ala Asp Glu Glu Asp Arg Phe Val
        515                 520                 525

Val Ala Gln Ala Asn Thr His Tyr Asp Glu Glu Gly Asn Ile Thr Asp
    530                 535                 540

Glu Thr Val Thr Val Arg Leu Lys Asp Gly Asp Ile Ala Met Val Gly
545                 550                 555                 560

Arg Asn Ala Val Asp Tyr Met Asp Val Ser Pro Arg Gln Met Val Ser
                565                 570                 575

Val Gly Thr Ala Met Ile Pro Phe Leu Glu His Asp Asp Ala Asn Arg
            580                 585                 590

Ala Leu Met Gly Ala Asn Met Gln Lys Gln Ala Val Pro Leu Ile Arg
        595                 600                 605

Ala Glu Ala Pro Phe Val Gly Thr Gly Met Glu Gln Arg Ala Ala Tyr
    610                 615                 620

Asp Ala Gly Asp Leu Val Ile Thr Pro Val Ala Gly Val Val Glu Asn
625                 630                 635                 640

Val Ser Ala Asp Phe Ile Thr Ile Met Ala Asp Asp Gly Lys Arg Glu
                645                 650                 655

Thr Tyr Leu Leu Arg Lys Phe Gln Arg Thr Asn Gln Gly Thr Ser Tyr
            660                 665                 670

Asn Gln Lys Pro Leu Val Asn Leu Gly Glu Arg Val Glu Ala Gly Gln
        675                 680                 685

Val Ile Ala Asp Gly Pro Gly Thr Phe Asn Gly Glu Met Ser Leu Gly
    690                 695                 700

Arg Asn Leu Leu Val Ala Phe Met Pro Trp Glu Gly His Asn Tyr Glu
705                 710                 715                 720

Asp Ala Ile Ile Leu Asn Gln Asn Ile Val Glu Gln Asp Ile Leu Thr
                725                 730                 735

Ser Ile His Ile Glu Glu His Glu Ile Asp Ala Arg Asp Thr Lys Leu
            740                 745                 750

Gly Ala Glu Glu Ile Thr Arg Asp Ile Pro Asn Val Ser Glu Glu Val
        755                 760                 765

Leu Lys Asp Leu Asp Asp Arg Gly Ile Val Arg Ile Gly Ala Asp Val
    770                 775                 780

Arg Asp Gly Asp Ile Leu Val Gly Lys Val Thr Pro Lys Gly Glu Thr
785                 790                 795                 800

Glu Leu Thr Pro Glu Glu Arg Leu Leu Arg Ala Ile Phe Gly Glu Lys
                805                 810                 815

Ala Arg Glu Val Arg Asp Thr Ser Met Lys Val Pro His Gly Glu Thr
            820                 825                 830

Gly Lys Val Ile Gly Val Arg His Phe Ser Arg Glu Asp Asp Asp Asp
        835                 840                 845

Leu Ala Pro Gly Val Asn Glu Met Ile Arg Ile Tyr Val Ala Gln Lys
    850                 855                 860

Arg Lys Ile Gln Asp Gly Asp Lys Leu Ala Gly Arg His Gly Asn Lys
865                 870                 875                 880

Gly Val Val Gly Lys Ile Leu Pro Gln Glu Asp Met Pro Phe Leu Pro
                885                 890                 895

Asp Gly Thr Pro Val Asp Ile Ile Leu Asn Thr His Gly Val Pro Arg
            900                 905                 910

Arg Met Asn Ile Gly Gln Val Leu Glu Thr His Leu Gly Trp Leu Ala
        915                 920                 925

Ser Ala Gly Trp Ser Val Asp Pro Glu Asp Pro Glu Asn Ala Glu Leu
    930                 935                 940

Val Lys Thr Leu Pro Ala Asp Leu Leu Glu Val Pro Ala Gly Ser Leu
945                 950                 955                 960

Thr Ala Thr Pro Val Phe Asp Gly Ala Ser Asn Glu Glu Leu Ala Gly
                965                 970                 975

Leu Leu Ala Asn Ser Arg Pro Asn Arg Asp Gly Asp Val Met Val Asn
            980                 985                 990

Ala Asp Gly Lys Ala Thr Leu Ile Asp Gly Arg Ser Gly Glu Pro Tyr
        995                 1000                1005

Pro Tyr Pro Val Ser Ile Gly Tyr Met Tyr Met Leu Lys Leu His
    1010                1015                1020

His Leu Val Asp Glu Lys Ile His Ala Arg Ser Thr Gly Pro Tyr
    1025                1030                1035

Ser Met Ile Thr Gln Gln Pro Leu Gly Gly Lys Ala Gln Phe Gly
    1040                1045                1050

Gly Gln Arg Phe Gly Glu Met Glu Val Trp Ala Met Gln Ala Tyr
    1055                1060                1065

Gly Ala Ala Tyr Thr Leu Gln Glu Leu Leu Thr Ile Lys Ser Asp
    1070                1075                1080

Asp Val Val Gly Arg Val Lys Val Tyr Glu Ala Ile Val Lys Gly
    1085                1090                1095

Glu Asn Ile Pro Asp Pro Gly Ile Pro Glu Ser Phe Lys Val Leu
    1100                1105                1110

Leu Lys Glu Leu Gln Ser Leu Cys Leu Asn Val Glu Val Leu Ser
    1115                1120                1125

Ala Asp Gly Thr Pro Met Glu Leu Ala Gly Asp Asp Asp Asp Phe
    1130                1135                1140

Asp Gln Ala Gly Ala Ser Leu Gly Ile Asn Leu Ser Arg Asp Glu
    1145                1150                1155

Arg Ser Asp Ala Asp Thr Ala
    1160                1165

<210> 7
<211> 1775
<212> DNA
<213> Corynebacterium glutamicum

<220>
<221> CDS
<222> (500)..(880)
<223> rpsL-Gen

<400> 7
cagctctaca agagtgtcta agtggcgggc attccatgct ttggaggagc gatcttcaaa 60

ttcctccaaa gtgagttgac ctcgggaaac agctgcagaa agttcatcca cgacttggtt 120

tcggttaagg tcagtggcga gcttctttgc tggttcgttt ccttgaggaa cagtcatggg 180

aaccattcta acaagggatt tggtgttttc tgcggctagc tgataatgtg aacggctgag 240

tcccactctt gtagttggga attgacggca cctcgcactc aagcgcggta tcgcccctgg 300

ttttccggga cgcggtggcg catgtttgca tttgatgagg ttgtccgtga catgtttggt 360

cgggccccaa aaagagcccc cttttttgcg tgtctggaca ctttttcaaa tccttcgcca 420

tcgacaagct cagccttcgt gttcgtcccc cgggcgtcac gtcagcagtt aaagaacaac 480

tccgaaataa ggatggttc atg cca act att cag cag ctg gtc cgt aag ggc 532
Met Pro Thr Ile Gln Gln Leu Val Arg Lys Gly
1               5                   10

cgc cac gat aag tcc gcc aag gtg gct acc gcg gca ctg aag ggt tcc 580
Arg His Asp Lys Ser Ala Lys Val Ala Thr Ala Ala Leu Lys Gly Ser
            15                  20                  25

cct cag cgt cgt ggc gta tgc acc cgt gtg tac acc acc acc cct aag 628
Pro Gln Arg Arg Gly Val Cys Thr Arg Val Tyr Thr Thr Thr Pro Lys
        30                  35                  40

aag cct aac tct gct ctt cgt aag gtc gct cgt gtg cgc ctt acc tcc 676
Lys Pro Asn Ser Ala Leu Arg Lys Val Ala Arg Val Arg Leu Thr Ser
    45                  50                  55

ggc atc gag gtt tcc gct tac atc cct ggt gag ggc cac aac ctg cag 724
Gly Ile Glu Val Ser Ala Tyr Ile Pro Gly Glu Gly His Asn Leu Gln
60                  65                  70                  75

gag cac tcc atg gtg ctc gtt cgc ggt ggt cgt gtt aag gac ctc cca 772
Glu His Ser Met Val Leu Val Arg Gly Gly Arg Val Lys Asp Leu Pro
                80                  85                  90

ggt gtc cgt tac aag atc gtc cgt ggc gca ctg gat acc cag ggt gtt 820
Gly Val Arg Tyr Lys Ile Val Arg Gly Ala Leu Asp Thr Gln Gly Val
            95                  100                 105

aag gac cgc aag cag gct cgt tcc ccg cta cgg cgc gaa gag ggg ata 868
Lys Asp Arg Lys Gln Ala Arg Ser Pro Leu Arg Arg Glu Glu Gly Ile
        110                 115                 120

att aaa aat gcg taaatcagca gctcctaagc gtccagtagt tcaggaccct 920
Ile Lys Asn Ala
    125

gtatacaagt ccgagctcgt tacccagctc gtaaacaaga tcctcatcgg tggcaagaag 980

tccaccgcag agcgcatcgt ctacggtgca ctcgagatct gccgtgagaa gaccggcacc 1040

gatccagtag gaaccctcga gaaggctctc ggcaacgtgc gtccagacct cgaagttcgt 1100

tcccgccgtg ttggtggcgc tacctaccag gtgccagtgg atgttcgccc agagcgcgca 1160

aacaccctcg cactgcgttg gttggtaacc ttcacccgtc agcgtcgtga gaacaccatg 1220

atcgagcgtc ttgcaaacga acttctggat gcagccaacg gccttggcgc ttccgtgaag 1280

cgtcgcgaag acacccacaa gatggcagag gccaaccgcg ccttcgctca ctaccgctgg 1340

tagtactgcc aagacatgaa agcccaatca cctttaagat caacgcctgc cggcgccctt 1400

cacatttgaa taagctggca gcctgcgttt cttcaaggcg actgggcttt tagtctcatt 1460

aatgcagttc accgctgtaa gatagctaaa tagaaacact gtttcggcag tgtgttacta 1520

aaaaatccat gtcacttgcc tcgagcgtgc tgcttgaatc gcaagttagt ggcaaaatgt 1580

aacaagagaa ttatccgtag gtgacaaact ttttaatact tgggtatctg tcatggatac 1640

cccggtaata aataagtgaa ttaccgtaac caacaagttg gggtaccact gtggcacaag 1700

aagtgcttaa ggatctaaac aaggtccgca acatcggcat catggcgcac atcgatgctg 1760

gtaagaccac gacca 1775

<210> 8
<211> 127
<212> PRT
<213> Corynebacterium glutamicum

<400> 8
Met Pro Thr Ile Gln Gln Leu Val Arg Lys Gly Arg His Asp Lys Ser
1               5                   10                  15

Ala Lys Val Ala Thr Ala Ala Leu Lys Gly Ser Pro Gln Arg Arg Gly
            20                  25                  30

Val Cys Thr Arg Val Tyr Thr Thr Thr Pro Lys Lys Pro Asn Ser Ala
        35                  40                  45

Leu Arg Lys Val Ala Arg Val Arg Leu Thr Ser Gly Ile Glu Val Ser
    50                  55                  60

Ala Tyr Ile Pro Gly Glu Gly His Asn Leu Gln Glu His Ser Met Val
65                  70                  75                  80

Leu Val Arg Gly Gly Arg Val Lys Asp Leu Pro Gly Val Arg Tyr Lys
                85                  90                  95

Ile Val Arg Gly Ala Leu Asp Thr Gln Gly Val Lys Asp Arg Lys Gln
            100                 105                 110

Ala Arg Ser Pro Leu Arg Arg Glu Glu Gly Ile Ile Lys Asn Ala
        115                 120                 125

<210> 9
<211> 24
<212> DNA
<213> Corynebacterium glutamicum

<220>
<221> misc_feature
<222> (1)..(24)
<223> Primer rpoB-1

<400> 9
acaatgtgac tcgtgatttt tggg 24

<210> 10
<211> 20
<212> DNA
<213> Corynebacterium glutamicum

<220>
<221> misc_feature
<222> (1)..(20)
<223> Primer rpoB3

<400> 10
ggaaacgtcc atgtaatcaa 20

<210> 11
<211> 20
<212> DNA
<213> Corynebacterium glutamicum

<220>
<221> misc_feature
<222> (1)..(20)
<223> Primer rpoB-4

<400> 11
aacacgcact acgacgaaga 20

<210> 12
<211> 20
<212> DNA
<213> Corynebacterium glutamicum

<220>
<221> misc_feature
<222> (1)..(20)
<223> Primer rpoB-2

<400> 12
cagcatcaag gtcgaagttc 20

## Beschreibung

Gegenstand der Erfindung sind für das rpoB-Gen kodierende Nukleotidsequenzen aus coryneformen Bakterien und ein Verfahren zur fermentativen Herstellung von Aminosäuren unter Verwendung von Bakterien, in denen das rpoB-Gen verstärkt wird.

### Stand der Technik

L-Aminosäuren, insbesondere L-Lysin, finden in der Humanmedizin und in der pharmazeutischen Industrie, in der Lebensmittelindustrie und ganz besonders in der Tierernährung, Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und Aminosäuren produzieren.

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die Aminosäure-Produktion untersucht.

### Aufgabe der Erfindung

Die Erfinder haben sich zur Aufgabe gestellt, neue Maßnahmen zur verbesserten fermentativen Herstellung von Aminosäuren bereitzustellen.

### Beschreibung der Erfindung

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan und L-Arginin gemeint. Besonders bevorzugt ist L-Lysin.

Wenn im folgenden L-Lysin oder Lysin erwähnt werden, sind damit nicht nur die Basen, sondern auch die Salze wie z.B. Lysin-Monohydrochlorid oder Lysin-Sulfat gemeint.

Gegenstand der Erfindung ist ein isoliertes Polynukleotid aus coryneformen Bakterien, enthaltend eine für das rpoB-Gen kodierende Polynukleotidsequenz, ausgewählt aus der Gruppe
a) Polynukleotid, das mindestens zu 70% identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c),
wobei das Polypeptid bevorzugt die Aktivität der β-Untereinheit der RNA-Polymerase B aufweist.

Gegenstand der Erfindung ist ebenfalls das oben genannte Polynukleotid, wobei es sich bevorzugt um eine replizierbare DNA handelt, enthaltend:
(i) die Nukleotidsequenz, gezeigt in SEQ ID No. 1, oder
(ii) mindestens eine Sequenz, die der Sequenz (i) innerhalb des Bereichs der Degeneration des genetischen Kodes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i), die die Aktivität des Proteins/Polypeptides nicht verändern

Ein weiterer Gegenstand der Erfindung sind schließlich Polynukleotide ausgewählt aus der Gruppe
a) Polynukleotide enthaltend mindestens 15 aufeinanderfolgende Nukleotide ausgewählt aus der Nukleotidsequenz von SEQ ID No. 1 zwischen den Positionen 1 und 701
b) Polynukleotide enthaltend mindestens 15 aufeinanderfolgende Nukleotide ausgewählt aus der Nukleotidsequenz von SEQ ID No. 1 zwischen den Positionen 702 und 4199
c) Polynukleotide enthaltend mindestens 15 aufeinanderfolgende Nukleotide ausgewählt aus der Nukleotidsequenz von SEQ ID No. 1 zwischen den Positionen 4200 und 5099.

Weitere Gegenstände sind
ein replizierbares Polynukleotid, insbesondere DNA, enthaltend die Nukleotidsequenz wie in SEQ ID No. 1 dargestellt;
ein Polynukleotid, das für ein Polypeptid kodiert, das die Aminosäuresequenz, wie in SEQ ID No. 2 dargestellt, enthält;
ein Vektor, enthaltend das erfindungsgemäße Polynukleotid, insbesondere Pendelvektor oder Plasmidvektor, und
coryneforme Bakterien, die den Vektor enthalten oder in denen das rpoB-Gen verstärkt ist.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank eines coryneformen Bakteriums, die das vollständige Gen oder Teile davon enthält, mit einer Sonde, die die Sequenz des erfindungsgemäßen Polynukleotids gemäß SEQ ID No.1 oder ein Fragment davon enthält und Isolierung der genannten Polynukleotidsequenz.

Polynukleotide, die die Sequenzen gemäß der Erfindung enthalten, sind als Hybridisierungssonden für RNA, cDNA und DNA geeignet, um Nukleinsäuren beziehungsweise Polynukleotide oder Gene in voller Länge zu isolieren, die für die β-Untereinheit der RNA-Polymerase B kodieren, oder um solche Nukleinsäuren beziehungsweise Polynukleotide oder Gene zu isolieren, die eine hohe Ähnlichkeit der Sequenz mit der des rpoB-Gens aufweisen. Sie können ebenso als Sonde auf sogenannte "arrays", "micro arrays" oder "DNA chips" aufgebracht werden, um die entsprechenden Polynukleotide oder hiervon abgeleitete Sequenzen wie z.B. RNA oder cDNA zu detektieren und zu bestimmen.

Polynukleotide, die die Sequenzen gemäß der Erfindung enthalten, sind weiterhin als Primer geeignet, mit deren Hilfe mit der Polymerase-Kettenreaktion (PCR) DNA von Genen hergestellt werden kann, die für die β-Untereinheit der RNA-Polymerase B kodieren.

Solche als Sonden oder Primer dienende Oligonukleotide, enthalten mindestens 25, 26, 27, 28, 29 oder 30, bevorzugt mindestens 20, 21, 22, 23 oder 24, ganz besonders bevorzugt mindestens 15, 16, 17, 18 oder 19 aufeinanderfolgende Nukleotide. Geeignet sind ebenfalls Oligonukleotide mit einer Länge von mindestens 31, 32, 33, 34, 35, 36, 37, 38, 39 oder 40, oder mindestens 41, 42, 43, 44, 45, 46, 47, 48, 49 oder 50 Nukleotiden. Gegebenenfalls sind auch Oligonukleotide mit einer Länge von mindestens 100, 150, 200, 250 oder 300 Nukleotiden geeignet.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Die Polynukleotide gemäß Erfindung schließen ein Polynukleotid gemäß SEQ ID No. 1 oder ein daraus hergestelltes Fragment und auch solche ein, die zu wenigstens besonders 70% bis 80%, bevorzugt zu wenigstens 81% bis 85%, besonders bevorzugt zu wenigstens 86% bis 90%, und ganz besonders bevorzugt zu wenigstens 91%, 93%, 95%, 97% oder 99% identisch sind mit dem Polynukleotid gemäß SEQ ID No. 1 oder eines daraus hergestellten Fragmentes.

Unter "Polypeptiden" versteht man Peptide oder Proteine, die zwei oder mehr über Peptidbindungen verbundene Aminosäuren enthalten.

Die Polypeptide gemäß Erfindung schließen ein Polypeptid gemäß SEQ ID No. 2, insbesondere solche mit der biologischen Aktivität der β-Untereinheit der RNA-Polymerase B und auch solche ein, die zu wenigstens 70% bis 80%, bevorzugt zu wenigstens 81% bis 85%, besonders bevorzugt zu wenigstens 86% bis 90%, und ganz besonders bevorzugt zu wenigstens 91%, 93%, 95%, 97% oder 99% identisch sind mit dem Polypeptid gemäß SEQ ID No. 2 und die genannte Aktivität aufweisen.

Die Erfindung betrifft weiterhin ein Verfahren zur fermentativen Herstellung von Aminosäuren, ausgewählt aus der Gruppe L-Asparagin, L-Threonin, L-Serin, L-Glutamat, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan und L-Arginin, unter Verwendung von coryneformen Bakterien, die insbesondere bereits Aminosäuren produzieren und in denen die für das rpoB-Gen kodierenden, bevorzugt endogenen, Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme bzw. Proteine in einem Mikroorganismus, die durch die entsprechende DNA kodiert werden, indem man beispielsweise die Kopienzahl des Gens bzw. der Gene erhöht, einen starken Promotor verwendet oder ein Gen oder Allel verwendet, das für ein entsprechendes Enzym bzw. Protein mit einer hohen Aktivität kodiert und gegebenenfalls diese Maßnahmen kombiniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise der Aktivität oder Konzentration des Proteins im Ausgangs-Mikroorganismus erhöht.

Die Mikroorganismen, die Gegenstand der vorliegenden Erfindung sind, können L-Aminosäuren aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum (C. glutamicum), sind besonders die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme, wie beispielsweise die L-Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464
Corynebacterium glutamicum DM58-1
Corynebacterium glutamicum DG52-5
Corynebacterium glutamicum DSM5714 und
Corynebacterium glutamicum DSM12866.
Das neue, für die β-Untereinheit der RNA-Polymerase B kodierende rpoB-Gen von C. glutamicum wurde isoliert.

Zur Isolierung des rpoB-Gens oder auch anderer Gene von C. glutamicum wird zunächst eine Genbank dieses Mikroorganismus in Escherichia coli (E. coli) angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990), oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde.

Börmann et al. (Molecular Microbiology 6(3), 317-326) (1992)) wiederum beschreiben eine Genbank von C. glutamicum ATCC13032 unter Verwendung des Cosmids pHC79 (Hohn und Collins, Gene 11, 291-298 (1980)).

Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z.B. bei Sanger et al. (Proceedings of the National Academy of Sciences of the United States of America, 74:5463-5467, 1977) beschrieben ist.

Die erhaltenen DNA-Sequenzen können dann mit bekannten Algorithmen bzw. Sequenzanalyse-Programmen wie z.B. dem von Staden (Nucleic Acids Research 14, 217-232(1986)), dem von Marck (Nucleic Acids Research 16, 1829-1836 (1988)) oder dem GCG-Programm von Butler (Methods of Biochemical Analysis 39, 74-97 (1998)) untersucht werden.

Die neue für das Gen rpoB kodierende DNA-Sequenz von C. glutamicum wurde gefunden, die als SEQ ID No. 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID No. 2 ist die sich ergebende Aminosäuresequenz des rpoB-Genproduktes dargestellt. Es ist bekannt, dass wirtseigene Enzyme die N-terminale Aminosäure Methionin bzw. Formylmethionin des gebildeten Proteins abspalten können.

Kodierende DNA-Sequenzen, die sich aus SEQ ID No. 1 durch die Degeneriertheit des genetischen Kodes ergeben, sind ebenfalls Bestandteil der Erfindung. In gleicher Weise sind DNA-Sequenzen, die mit SEQ ID No. 1 oder Teilen von SEQ ID No. 1 hybridisieren, Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als "Sinnmutationen" ("sense mutations") bekannt, die zu keiner grundsätzlichen Veränderung der Aktivität des Proteins führen, d.h. funktionsneutral sind. Derartige Mutationen werden unter anderem auch als neutrale Substitutionen bezeichnet. Weiterhin ist bekannt, dass Änderungen am N-und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie.
Aminosäuresequenzen, die sich in entsprechender Weise aus SEQ ID No. 2 ergeben, sind ebenfalls Bestandteil der Erfindung.

In gleicher Weise sind DNA-Sequenzen, die mit SEQ ID No. 1 oder Teilen von SEQ ID No. 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID No. 1 ergeben. Derartige Oligonukleotide haben typischerweise eine Länge von mindestens 15 Nukleotiden.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflußt bzw. bestimmt wird. Die Hybridisierungsreaktion wird vorzugsweise bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C eingestellt wird. Es ist gegebenenfalls möglich die Salzkonzentration bis auf 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90% bis 95% Identität zur Sequenz der eingesetzten Sonde besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Anleitungen zur Amplifikation von DNA-Sequenzen mit Hilfe der Polymerase-Kettenreaktion (PCR) findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994).

Es wurde gefunden, dass coryneforme Bakterien nach Verstärkung des rpoB-Gens in verbesserter Weise Aminosäuren produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen Aminosäure-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Zur Verstärkung wurde das erfindungsgemäße rpoB-Gen beispielhaft mit Hilfe von episomalen Plasmiden überexprimiert. Als Plasmide eignen sich solche, die in coryneformen Bakterien repliziert werden. Zahlreiche bekannte Plasmidvektoren wie z.B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z.B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise PSUP301 (Simon et al., Bio/Technology 1, 784-791 (1983)), pKl8mob oder pK19mob (Schäfer et al., Gene 145, 69-73 (1994)), pGEM-T (Promega Corporation, Madison, WI, USA), pCR2.1-TOPO (Shuman (1994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)), pEM1 (Schrumpf et al, 1991, Journal of Bacteriology 173:4510-4516) oder pBGS8 (Spratt et al.,1986, Gene 41: 337-342) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses enthält der resultierende Stamm mindestens zwei Kopien des betreffenden Gens.

Es wurde weiterhin gefunden, dass der Austausch von Aminosäuren, insbesondere in den Abschnitten zwischen Position 1 bis 10, 190 bis 200 und 420 bis 450 in der Aminosäuresequenz der β-Untereinheit der RNA-Polymerase B dargestellt in SEQ ID No. 2, die Lysinproduktion coryneformer Bakterien verbessern.

Es wurde auch gefunden, dass der Austausch von Aminosäuren an einer oder mehreren Positionen ausgewählt aus der Gruppe a) Position 1 bis 10, b) Position 190 bis 200 und c) Position 420 bis 450 in der SEQ ID No. 2 gleichzeitig erfolgen kann.

In dem Bereich zwischen Position 1 bis 10 wird der Austausch von L-Prolin an der Position 5 gegen L-Leucin, L-Isoleucin oder L-Valin bevorzugt.

In dem Bereich zwischen Position 190 bis 200 wird der Austausch von L-Serin an der Position 196 gegen L-Phenylalanin oder L-Tyrosin bevorzugt.

In dem Bereich zwischen 420 bis 450 werden folgende Austausche bevorzugt: Austausch von L-Leucin an der Position 424 gegen L-Prolin oder L-Arginin, Austausch von L-Serin an der Position 425 gegen L-Threonin oder L-Alanin, Austausch von L-Glutamin an Position 426 gegen L-Leucin oder L-Lysin, Austausch von L-Asparaginsäure an Position 429 gegen L-Isoleucin, L-Valin oder L-Leucin, Austausch von L-Histidin an Position 439 gegen jede andere proteinogene Aminosäure ausgenommen L-Histidin, wird der Austausch von L-Serin an Position 444 gegen L-Leucin, L-Tyrosin oder L-Tryptophan und Austausch von L-Leucin an Position 446 gegen L-Prolin oder L-Isoleucin.

Ganz besonders bevorzugt, werden ein oder mehrere Aminosäureaustausche ausgewählt aus der Gruppe: L-Prolin an Position 5 gegen L-Leucin, L-Serin an Position 196 gegen L-Phenylalanin, L-Aspartat an Position 429 gegen L-Valin und L-Histidin an Position 439 gegen L-Tyrosin.

In SEQ ID No. 3 ist die Basensequenz des in Stamm DM1547 enthaltenen Allels rpoB-1547 dargestellt. Das rpoB-1547 Allel kodiert für ein Protein, dessen Aminosäuresequenz in SEQ ID No. 4 dargestellt ist. Das Protein enthält an Position 5 L-Leucin, an Position 196 L-Phenylalanin und an Position 429 L-Valin. Die DNA Sequenz des rpoB-1547 Allels (SEQ ID No. 3) enthält folgende Basenaustausche gegenüber dem rpoB Wildtypgen (SEQ ID No. 1) : Thymin an Position 715 anstelle von Cytosin, Thymin an Position 1288 anstelle von Cytosin und Thymin an Position 1987 anstelle von Adenin.

In SEQ ID No. 5 ist die Basensequenz des in Stamm DM1546 enthaltenen Allels rpoB-1546 dargestellt. Das rpoB-1546 Allel kodiert für ein Protein, dessen Aminosäuresequenz in SEQ ID No. 6 dargestellt ist. Das Protein enthält an Position 439 L-Tyrosin. Die DNA Sequenz des rpoB-1546 Allels (SEQ ID No. 5) enthält folgende Basenaustausche gegenüber dem rpoB Wildtypgen (SEQ ID No. 1): Thymin an Position 2016 anstelle von Cytosin.

Für die Mutagenese können klassische Mutageneseverfahren unter Verwendung mutagener Stoffe wie beispielsweise N-Methyl-N'-Nitro-N-Nitrosoguanidin oder ultraviolettes Licht verwendet werden. Weiterhin können für die Mutagenese invitro Methoden wie beispielsweise eine Behandlung mit Hydroxylamin (Miller, J. H.: A Short Course in Bacterial Genetics. A Laboratory Manual and Handbook for Escherichia coli and Related Bacteria, Cold Spring Harbor Laboratory Press, Cold Spring Harbor, 1992) oder mutagene Oligonukleotide (T. A. Brown: Gentechnologie für Einsteiger, Spektrum Akademischer Verlag, Heidelberg, 1993) oder die Polymerasenkettenreaktion (PCR), wie sie im Handbuch von Newton und Graham (PCR, Spektrum Akademischer Verlag, Heidelberg, 1994) beschrieben ist, verwendet werden.

Die erfindungsgemäßen rpoB-Allele können unter anderem auch durch das Verfahren des Genaustausches ("gene replacement"), wie es bei Schwarzer und Pühler (Bio/Technology 9, 84-87 (1991)) oder Peters-Wendisch et al. (Microbiology 144, 915 - 927 (1998)) beschrieben ist, in geeignete Stämme überführt werden. Das entsprechende rpsL-Allel wird hierbei in einen für C. glutamicum nicht replikativen Vektor wie beispielsweise pK18mobsacB oder pK19mobsacB (Jäger et al., Journal of Bacteriology 174: 5462-65 (1992)) oder pCR®Blunt (Firma Invitrogen, Groningen, Niederlande; Bernard et al., Journal of Molecular Biology, 234: 534-541 (1993)) kloniert und dieser anschließend durch Transformation oder Konjugation in den gewünschten Wirt von C. glutamicum überführt. Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation.

Zusätzlich kann es für die Produktion von L-Aminosäuren vorteilhaft sein, neben dem rpoB-Gen eines oder mehrere Enzyme des jeweiligen Biosyntheseweges, der Glykolyse, der Anaplerotik, des Zitronensäure-Zyklus, des Pentosephosphat-Zyklus, des Aminosäure-Exports und gegebenenfalls regulatorische Proteine zu verstärken, insbesondere überzuexprimieren. Die Verwendung endogener Gene wird im allgemeinen bevorzugt.

Unter "endogenen Genen" oder "endogenen Nukleotidsequenzen" versteht man die in der Population einer Art vorhandenen Gene beziehungsweise Nukleotidsequenzen und deren Allele.

So kann für die Herstellung von L-Lysin zusätzlich zur Verstärkung des rpoB-Gens eines oder mehrere der Gene, ausgewählt aus der Gruppe
- das für die Dihydrodipicolinat-Synthase kodierende Gen dapA (EP-B 0 197 335),
- das für die Glyceraldehyd-3-Phosphat-Dehydrogenase kodierende Gen gap (Eikmanns (1992), Journal of Bacteriology 174:6076-6086),
- das für die Triosephosphat-Isomerase kodierende Gen tpi (Eikmanns (1992), Journal of Bacteriology 174:6076-6086),
- das für die 3-Phosphoglycerat-Kinase kodierende Gen pgk (Eikmanns (1992), Journal of Bacteriology 174:6076-6086),
- das für die Glucose-6-Phosphat-Dehydrogenase kodierende Gen zwf (JP-A-09224661),
- das für die Pyruvat-Carboxylase kodierende Gen pyc (DE-A-198 31 609),
- das für die Malat-Chinon-Oxidoreduktase kodierende Gen mqo (Molenaar et al., European Journal of Biochemistry 254, 395-403 (1998)),
- das für eine feed-back resistente Aspartatkinase kodierende Gen lysC (Kalinowski et al., Molecular Microbiologie 5(5), 1197-204 (1991)),
- das für das Lysin-Export-Protein kodierende Gen lysE (DE-A-195 48 222),
- das für das Zwa1-Protein kodierende Gen zwa1 (DE: 19959328.0, DSM 13115), und
- das für das ribosomale Protein S12 kodierende rpsL-Gen dargestellt in SEQ ID No. 7 und 8
verstärkt, insbesondere überexprimiert werden.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt.

Weiterhin kann es für die Produktion von L-Aminosäuren vorteilhaft sein, zusätzlich zur Verstärkung des rpoB-Gens eines oder mehrere Gene, ausgewählt aus der Gruppe
- das für die Phosphoenolpyruvat-Carboxykinase kodierende Gen pck (DE 199 50 409.1; DSM 13047),
- das für die Glucose-6-Phosphat-Isomerase kodierende Gen pgi (US 09/396,478; DSM 12969),
- das für die Pyruvat-Oxidase kodierende Gen poxB (DE: 1995 1975.7; DSM 13114),
- das für das Zwa2-Protein kodierende Gen zwa2 (DE: 19959327.2, DSM 13113)
abzuschwächen, insbesondere die Expression zu verringern.

Weiterhin kann es für die Produktion von Aminosäuren vorteilhaft sein, neben der Verstärkung des rpoB-Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die erfindungsgemäß hergestellten Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von Aminosäuren kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Lactose, Fructose, Maltose, Melasse, Stärke und Cellulose, Öle und Fette wie z.B. Sojaöl, Sonnenblumenöl, Erdnußöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muss weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B. Antibiotika hinzugefügt werden. Um aerobe Bedingungen aufrechtzuerhalten, werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Ionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Reinkulturen folgender Mikroorganismen wurden bei der Deutschen Sammlung für Mikroorganismen und Zellkulturen (DSMZ, Braunschweig, Deutschland)am 16. Januar 2001 gemäß Budapester Vertrag hinterlegt:
- Corynebacterium glutamicum Stamm DM1546 als DSM 13993
- Corynebacterium glutamicum Stamm DM1547 als DSM 13994

Das erfindungsgemäße Verfahren dient zur fermentativen Herstellung von Aminosäuren.

Die vorliegende Erfindung wird im folgenden anhand von Ausführungsbeispielen näher erläutert.

Die Isolierung von Plasmid-DNA aus Escherichia coli sowie alle Techniken zur Restriktion, Klenow- und alkalische Phosphatasebehandlung wurden nach Sambrook et al. (Molecular Cloning. A Laboratory Manual (1989) Cold Spring Harbor Laboratory Press, Cold Spring Harbor, NY, USA) durchgeführt. Methoden zur Transformation von Escherichia coli sind ebenfalls in diesem Handbuch beschrieben.

Die Zusammensetzung gängiger Nährmedien wie LB- oder TY-Medium kann ebenfalls dem Handbuch von Sambrook et al. entnommen werden.

### Beispiel 1

### Herstellung einer genomischen Cosmid-Genbank aus Corynebacterium glutamicum ATCC 13032

Chromosomale DNA aus Corynebacterium glutamicum ATCC 13032 wird wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente werden mit shrimp alkalischer Phosphatase (Roche Diagnostics GmbH, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCos1 Cosmid Vektor Kit, Code no. 251301) wird mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert.

Anschließend wird die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wird mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wird anschließend mit Hilfe des Gigapack II XL Packing Extracts (Stratagene, La Jolla, USA, Produktbeschreibung Gigapack II XL Packing Extract, Code no. 200217) in Phagen verpackt.

Zur Infektion des E. coli Stammes NM554 (Raleigh et al. 1988, Nucleic Acid Research 16:1563-1575) werden die Zellen in 10 mM MgSO₄ aufgenommen und mit einem Aliquot der Phagensuspension vermischt. Infektion und Titerung der Cosmidbank werden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 mg/l Ampicillin ausplattiert werden. Nach Inkubation über Nacht bei 37°C werden rekombinante Einzelklone selektioniert.

### Beispiel 2

### Isolierung und Sequenzierung des rpoB-Gens

Die Cosmid-DNA einer Einzelkolonie wird mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente werden mit shrimp alkalischer Phosphatase (Roche Diagnostics GmbH, Mannheim, Deutschland, Produktbeschreibung SAP, Product No. 1758250) dephosphoryliert. Nach gelelektrophoretischer Auftrennung erfolgt die Isolierung der Cosmidfragmente im Größenbereich von 1500 bis 2000 bp mit dem QiaExII Gel Extraction Kit (Product No. 20021, Qiagen, Hilden, Germany).

Die DNA des Sequenziervektors pZero-1, bezogen von der Firma Invitrogen (Groningen, Niederlande, Produktbeschreibung Zero Background Cloning Kit, Product No. K2500-01), wird mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wird wie von Sambrook et al. (1989, Molecular Cloning: A Laboratory Manual, Cold Spring Harbor) beschrieben durchgeführt, wobei das DNA-Gemisch mit T4-Ligase (Pharmacia Biotech, Freiburg, Deutschland) über Nacht inkubiert wird. Dieses Ligationsgemisch wird anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 mg/l Zeocin ausplattiert.

Die Plasmidpräparation der rekombinanten Klone erfolgt mit dem Biorobot 9600 (Product No. 900200, Qiagen, Hilden, Deutschland). Die Sequenzierung erfolgt nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wird der "RR dRhodamin Terminator Cycle Sequencing Kit" von PE Applied Biosystems (Product No. 403044, Weiterstadt, Deutschland) verwendet. Die gelelektrophoretische Auftrennung und Analyse der Sequenzierreaktion erfolgt in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten werden anschließend unter Anwendung des Staden-Programpakets (1986, Nucleic Acids Research, 14:217-231) Version 97-0 prozessiert. Die Einzelsequenzen der pZero1-Derivate werden zu einem zusammenhängenden Contig assembliert. Die computergestützte Kodierbereichsanalyse wird mit dem Programm XNIP (Staden, 1986, Nucleic Acids Research, 14:217-231) angefertigt.

Die erhaltene Nukleotidsequenz ist in SEQ ID No. 1 dargestellt. Die Analyse der Nukleotidsequenz ergibt ein offenes Leseraster von 3498 Basenpaaren, welches als rpoB-Gen bezeichnet wird. Das rpoB-Gen kodiert für ein Protein von 1165 Aminosäuren.

### Beispiel 3

### Herstellung und Selektion des Stammes DM1546

Ausgangsstamm für die Herstellung des Stammes DM1546 ist der Lysin produzierende C. glutamicum Stamm DM678, der durch mehrfache Mutagenese und Screening aus dem Stamm ATCC13032 abgeleitet wurde. Er besitzt eine partielle Threonin-Bedürftigkeit und ist Methionin-sensitiv. Der Stamm DM678 wurde in Form einer Reinkultur am 10. Juni 1999 bei der DSMZ (Deutsche Sammlung für Mikroorganismen und Zellkulturen, Braunschweig, Deutschland) gemäß Budapester Vertrag als DSM12866 hinterlegt.

Mit dem Stamm DM678 wird eine erneute ungerichtete Mutagenese unter Verwendung von N-Methyl-N'-Nitro-N-Nitrosoguanidin (Miller, J. H.: A Short Course in Bacterial Genetics. A Laboratory Manual and Handbook for Escherichia coli and Related Bacteria, Cold Spring Harbor Laboratory Press, Cold Spring Harbor, 1992) durchgeführt.

Mehr als hundert der auf diese Weise erhaltenen Mutanten und der Ausgangsstamm DM678 werden in einem zur Produktion von Lysin geeigneten Nährmedium kultiviert und der Lysingehalt im Kulturüberstand bestimmt.

Dazu werden die Stämme zunächst auf Agarplatte für 24 Stunden bei 33°C inkubiert. Ausgehend von diesen Agarplattenkulturen wird je eine Vorkultur angeimpft (10 ml Medium im 100 ml Erlenmeyerkolben). Als Medium für die Vorkulturen wird das Medium MM verwendet. Die Vorkulturen werden 24 Stunden bei 33°C bei 240 rpm auf dem Schüttler inkubiert. Von diesen Vorkulturen wird je eine Hauptkultur angeimpft, so dass die Anfangs-OD (660 nm) der Hauptkulturen 0,1 OD beträgt. Für die Hauptkulturen wird ebenfalls das Medium MM verwendet.

| Medium MM | |
|---|---|
| CSL | 5 g/l |
| MOPS | 20 g/l |
| Glucose (getrennt autoklaviert) | 50 g/l |
| Salze: | |
| (NH₄)₂SO₄ | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung werden mit Ammoniakwasser auf pH 7 eingestellt und autoklaviert. Anschließend werden die sterilen Substrat- und Vitaminlösungen sowie das trocken autoklavierte CaCO₃ zugesetzt.

Die Kultivierung erfolgt in 10 ml Volumen in einem 100 ml Erlenmeyerkolben mit Schikanen. Die Kultivierung erfolgt bei 33°C und 80% Luftfeuchte.

Nach 72 Stunden wird die OD der Kulturen bei einer Meßwellenlänge von 660 nm mit dem Biomek 1000 (Beckmann Instruments GmbH, München) ermittelt. Die gebildete Lysinmenge wird mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

Die Lysin-Produktion einer Mutante, hebt sich besonders von der der anderen getesteten Stämme ab. Dieser Stamm wurde als DM1546 bezeichnet. Das Ergebnis des Versuches ist in Tabelle 1 dargestellt.

**Tabelle 1**

| Stamm | OD (660 nm) | Lysin-HCl g/l |
|---|---|---|
| DM678 | 9,5 | 14,94 |
| DM1546 | 10,7 | 16,66 |

### Beispiel 4

### Sequenzierung des rpoB-Gens aus DM1546

Aus dem in Beispiel 3 erhaltenen Stamm DM1546 wird mit den üblichen Methoden (Eikmanns et al., Microbiology 140: 1817 - 1828 (1994)) chromosomale DNA isoliert. Aufgrund der Länge des rpoB-Gens ( Kodierbereich 3498 bp, siehe SEQ ID No. 1) werden mit Hilfe der Polymerase-Kettenreaktion zwei überlappende DNA-Abschnitte amplifiziert, welche zusammen das rpoB-Gen bzw. Allel vollständig enthalten. Aufgrund der aus Beispiel 2 für C. glutamicum bekannten Sequenz des rpoB-Gens werden folgende Primer-Oligonukleotide für die PCR ausgewählt:

rpoB-1 (SEQ ID No. 9):
5` aca atg tga ctc gtg att ttt ggg 3`

rpoB-3 (SEQ ID No. 10):
5` gga aac gtc cat gta atc aa 3`

rpoB-4 (SEQ ID No. 11):
5` aac acg cac tac gac gaa ga 3`

rpoB-2 (SEQ ID No. 12):
5` cag cat caa ggt cga agt tc 3`

Die dargestellten Primer werden von der Firma MWG Biotech(Ebersberg, Deutschland) synthetisiert und nach der Standard-PCR-Methode von Innis et al. (PCR Protocols. A Guide to Methods and Applications, 1990, Academic Press) die PCR Reaktion durchgeführt. Die Primer rpoB-1 und rpoB-3 ermöglichen die Amplifizierung eines ca. 2,41 kb langen DNA-Abschnittes, welcher den 5'-terminalen Bereich des rpoB-Allels enthält, und die Primer rpoB-4 und rpoB-2 die Amplifizierung eines ca. 2,8 kb langen DNA-Abschnittes, welcher den 3'-terminalen Bereich des rpoB-Allels enthält.

Die amplifizierten DNA-Fragmente von ca. 2,41 kb und ca. 2,8 kb Länge werden durch Elektrophorese in einem 0,8%igen Agarosegel identifiziert, aus dem Gel isoliert und mit den üblichen Methoden aufgereinigt (QIAquick Gel Extraction Kit, Qiagen, Hilden).

Die Nukleotidsequenz der amplifizierten DNA-Fragmente bzw. PCR-Produkte wird von der Firma MWG Biotech (Ebersberg, Deutschland) durch Sequenzierung ermittelt. Die Sequenz der PCR-Produkte überlappt im mittleren Bereich um 114 bp. Durch Zusammensetzen beider Sequenzen wird die Gesamtsequenz des rpoB-Allels des Stammes DM1546 einschließlich der upstream- und downstream-Region erhalten, die Sequenz ist in SEQ ID No. 5 dargestellt. Die sich mit Hilfe des Programmes Patentin ergebende Aminosäuresequenz der dazugehörigen β-Untereinheit der RNA-Polymerase B ist in der SEQ ID No. 6 dargestellt.

An Position 1315 der Nukleotidsequenz der Kodierregion (CDS) des rpoB-Allels von Stamm DM1546, also an Position 2016 der in SEQ ID No. 5 dargestellten Nukleotidsequenz, befindet sich die Base Thymin. An der entsprechenden Position des Wildtypgens (SEQ ID No. 1) befindet sich die Base Cytosin.

An Position 439 der Aminosäuresequenz der β-Untereinheit der RNA-Polymerase B von Stamm DM1546 befindet sich die Aminosäure L-Tyrosin (SEQ ID No. 6). An der entsprechenden Position des Wildtyp-Proteins befindet sich die Aminosäure L-Histidin (SEQ ID No. 2).

### Beispiel 5

### Sequenzierung des rpoB-Gens aus DM1547

Der Corynebacterium glutamicum Stamm DM1547 wurde durch mehrfache, ungerichtete Mutagenese, Selektion und Mutantenauswahl aus C. glutamicum ATCC13032 hergestellt. Der Stamm ist resistent gegen das Lysin-Analogon S-(2-Aminoethyl)-L-Cystein.

Aus Stamm DM1547 wird mit den üblichen Methoden (Eikmanns et al., Microbiology 140: 1817 - 1828 (1994)) chromosomale DNA isoliert. Aufgrund der Länge des rpoB-Gens (3498 bp, siehe SEQ ID No. 1) werden mit Hilfe der Polymerase-Kettenreaktion zwei überlappende DNA-Abschnitte amplifiziert, welche zusammen das rpoB-Gen bzw. Allel vollständig enthalten. Aufgrund der aus Beispiel 2 für C. glutamicum bekannten Sequenz des rpoB-Gens werden folgende Primer-Oligonukleotide für die PCR ausgewählt:

rpoB-1 (SEQ ID No. 9):
5` aca atg tga ctc gtg att ttt ggg 3`

rpoB-3 (SEQ ID No. 10):
5` gga aac gtc cat gta atc aa 3`

rpoB-4 (SEQ ID No. 11):
5` aac acg cac tac gac gaa ga 3`

rpoB-2 (SEQ ID No. 12):
5` cag cat caa ggt cga agt tc 3`

Die dargestellten Primer werden von der Firma MWG Biotech(Ebersberg, Deutschland) synthetisiert und nach der Standard-PCR-Methode von Innis et al. (PCR Protocols. A Guide to Methods and Applications, 1990, Academic Press) die PCR Reaktion durchgeführt. Die Primer rpoB-1 und rpoB-3 ermöglichen die Amplifizierung eines ca. 2,41 kb langen DNA-Abschnittes, welcher den 5'-terminalen Bereich des rpoB-Allels enthält, und die Primer rpoB-4 und rpoB-2 die Amplifizierung eines ca. 2,8 kb langen DNA-Abschnittes, welcher den 3'-terminalen Bereich des rpoB-Allels enthält.

Die amplifizierten DNA-Fragmente von ca. 2,41 kb und ca. 2,8 kb Länge werden durch Elektrophorese in einem 0,8%igen Agarosegel identifiziert, aus dem Gel isoliert und mit den üblichen Methoden aufgereinigt (QIAquick Gel Extraction Kit, Qiagen, Hilden).

Die Nukleotidsequenz der amplifizierten DNA-Fragmente bzw. PCR-Produkte wird von der Firma MWG Biotech (Ebersberg, Deutschland) durch Sequenzierung ermittelt. Die Sequenz der PCR-Produkte überlappt im mittleren Bereich um 114 bp. Durch Zusammensetzten beider Sequenzen wird die Gesamtsequenz des rpoB-Allels des Stammes DM1547 einschließlich der upstream- und downstream-Region erhalten, die Sequenz ist in SEQ ID No. 3 dargestellt. Die sich mit Hilfe des Programmes Patentin ergebende Aminosäuresequenz der dazugehörigen β-Untereinheit der RNA-Polymerase B ist in der SEQ ID No. 4 dargestellt.

Die Sequenz des rpoB-Allels von Stamm DM1547 (SEQ ID No. 3) enthält folgende Basenaustausche gegenüber dem rpoB-Wildtypgen (SEQ ID No. 1): an Position 14 des Kodierbereichs (CDS), also an Position 715 der SEQ ID No. 3, befindet sich die Base Thymin anstelle von Cytosin, an Position 587 des Kodierbereichs, also an Position 1288 der SEQ ID No. 3, befindet sich die Base Thymin anstelle von Cytosin und an Position 1286 des Kodierbereichs, also an Position 1987 der SEQ ID No. 3, befindet sich die Base Thymin anstelle von Adenin.

An Position 5 der Aminosäuresequenz der β-Untereinheit der RNA-Polymerase B von Stamm DM1547 befindet sich die Aminosäure L-Leucin, an Position 196 die Aminosäure L-Phenylalanin und an Position 429 die Aminosäure L-Valin (SEQ ID No. 4). Das Wildtyp-Protein enthält an Position 5 die Aminosäure L-Prolin, an Position 196 die Aminosäure L-Serin und an Position 429 die Aminosäure L-Asparaginsäure (SEQ ID No. 2).

## Patentansprüche

1. Isoliertes Polynukleotid aus coryneformen Bakterien, enthaltend eine für das rpoB-Gen kodierende Polynukleotidsequenz, ausgewählt aus der Gruppe
a) Polynukleotid, das mindestens zu 70% identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c)
wobei das Polypeptid bevorzugt die Aktivität der β-Untereinheit der RNA-Polymerase B aufweist.

2. Polynukleotid gemäß Anspruch 1, wobei das Polynukleotid eine in coryneformen Bakterien replizierbare, bevorzugt rekombinante DNA ist.

3. Polynukleotid gemäß Anspruch 1, wobei das Polynukleotid eine RNA ist.

4. Polynukleotid gemäß Anspruch 2, enthaltend die Nukleinsäuresequenz wie in SEQ ID No. 1 dargestellt.

5. Replizierbare DNA gemäß Anspruch 2, enthaltend
(i) die Nukleotidsequenz, gezeigt in SEQ ID No. 1, oder
(ii) mindestens eine Sequenz, die der Sequenz (i) innerhalb des Bereichs der Degeneration des genetischen Kodes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

6. Replizierbare DNA gemäß Anspruch 5, **dadurch gekennzeichnet, dass** die Hybridisierung unter einer Stringenz entsprechend höchstens 2x SSC durchgeführt wird.

7. Polynukleotidsequenz gemäß Anspruch 1, die für ein Polypeptid kodiert, das die in SEQ ID No. 2 dargestellte Aminosäuresequenz enthält.

8. Coryneforme Bakterien, in denen das rpoB-Gen verstärkt, insbesondere überexprimiert wird.

9. Corynebacterium glutamicum Stamm DM1546 hinterlegt als DSM 13993 bei der Deutschen Sammlung für Mikroorganismen und Zellkulturen (DSMZ, Braunschweig, Deutschland).

10. Corynebacterium glutamicum Stamm DM1547 hinterlegt als DSM 13994 bei der Deutschen Sammlung für Mikroorganismen und Zellkulturen (DSMZ, Braunschweig, Deutschland).

11. Verfahren zur fermentativen Herstellung von L-Aminosäuren, insbesondere L-Lysin, **dadurch gekennzeichnet, dass** man folgende Schritte durchführt:
a) Fermentation der die gewünschte L-Aminosäure produzierenden coryneformen Bakterien, in denen man zumindest das rpoB-Gen oder dafür kodierende Nukleotidsequenzen verstärkt, insbesondere überexprimiert;
b) Anreicherung der L-Aminosäure im Medium oder in den Zellen der Bakterien, und
c) Isolierung der L-Aminosäure.

12. Verfahren gemäß Anspruch 11, **dadurch gekennzeichnet, dass** man Bakterien einsetzt, in denen man zusätzlich weitere Gene des Biosyntheseweges der gewünschten L-Aminosäure verstärkt.

13. Verfahren gemäß Anspruch 11, **dadurch gekennzeichnet, dass** man Bakterien einsetzt, in denen die Stoffwechselwege zumindest teilweise ausgeschaltet sind, die die Bildung der gewünschten L-Aminosäure verringern.

14. Verfahren gemäß Anspruch 11, **dadurch gekennzeichnet, dass** man einen mit einem Plasmidvektor transformierten Stamm einsetzt, und der Plasmidvektor die für das rpoB-Gen kodierende Nukleotidsequenz trägt.

15. Verfahren gemäß Anspruch 11, **dadurch gekennzeichnet, dass** man die Expression des (der) Polynukleotids (e), das (die) für das rpoB-Gen kodiert (kodieren) verstärkt, insbesondere überexprimiert.

16. Verfahren gemäß Anspruch 11, **dadurch gekennzeichnet, dass** man die regulatorischen/katalytischen Eigenschaften des Polypeptids (Enzymprotein) erhöht, für das das Polynukleotid rpoB kodiert.

17. Verfahren gemäß Anspruch 11, **dadurch gekennzeichnet, dass** man zur Herstellung von L-Aminosäuren coryneforme Mikroorganismen fermentiert, in denen man gleichzeitig eines oder mehrere der Gene, ausgewählt aus der Gruppe
17.1 das für die Dihydrodipicolinat-Synthase kodierende Gen dapA,
17.2 das für die Glyceraldehyd-3-Phosphat-Dehydrogenase kodierende Gen gap,
17.3 das für die Triosephosphat-Isomerase kodierende Gen tpi,
17.4 das für die 3-Phosphoglycerat-Kinase kodierende Gen pgk,
17.5 das für die Glucose-6-Phosphat-Dehydrogenase kodierende Gen zwf,
17.6 das für die Pyruvat-Carboxylase kodierende Gen pyc,
17.7 das für die Malat-Chinon-Oxidoreduktase kodierende Gen mqo,
17.8 das für eine feed-back resistente Aspartatkinase kodierende Gen lysC,
17.9 das für das Lysin-Export-Protein kodierende Gen lysE,
17.10 das für das Zwa1-Protein kodierende Gen zwa1,
17.11 das für das ribosomale Protein S12 kodierende rpsL-Gen
verstärkt bzw. überexprimiert.

18. Verfahren gemäß Anspruch 11, **dadurch gekennzeichnet, dass** man zur Herstellung von L-Aminosäuren coryneforme Mikroorganismen fermentiert, in denen man gleichzeitig eines oder mehrere der Gene, ausgewählt aus der Gruppe
18.1 das für die Phosphoenolpyruvat-Carboxykinase kodierende Gen pck,
18.2 das für die Glucose-6-Phosphat Isomerase kodierende Gen pgi,
18.3 das für die Pyruvat-Oxidase kodierende Gen poxB,
18.4 das für das Zwa2-Protein kodierende Gen zwa2
abschwächt.

19. Coryneforme Bakterien, die einen Vektor enthalten, der ein Polynukleotid gemäß Anspruch 1 trägt.

20. Verfahren gemäß einem oder mehreren der Ansprüche 11-18, **dadurch gekennzeichnet, dass** man Mikroorganismen der Art Corynebacterium glutamicum einsetzt.

21. Verfahren zum Auffinden von RNA, cDNA und DNA, um Nukleinsäuren, beziehungsweise Polynukleotide oder Gene zu isolieren, die für die β-Untereinheit der RNA-Polymerase B kodieren oder eine hohe Ähnlichkeit mit der Sequenz des rpoB-Gens aufweisen, **dadurch gekennzeichnet, dass** man das Polynukleotid, enthaltend die Polynukleotidsequenzen gemäß den Ansprüchen 1, 2, 3 oder 4, als Hybridisierungssonden einsetzt.

22. Verfahren gemäß Anspruch 21, **dadurch gekennzeichnet, dass** man arrays, micro arrays oder DNA-chips einsetzt.

23. Aus coryneformen Bakterien stammende DNA, kodierend für β-Untereinheiten der RNA-Polymerase B, wobei die zugehörigen Aminosäuresequenzen zwischen den Positionen 1 bis 10 in der SEQ ID No. 2 durch Aminosäureaustausch verändert sind.

24. DNA gemäß Anspruch 23, **dadurch gekennzeichnet, dass** diese für β-Untereinheiten der RNA-Polymerase B kodieren, wobei die zugehörigen Aminosäuresequenzen an Position 5 in der SEQ ID No. 2 L-Leucin, L-Isoleucin oder L-Valin enthalten.

25. Aus coryneformen Bakterien stammende DNA, kodierend für β-Untereinheiten der RNA-Polymerase B, wobei die zugehörigen Aminosäuresequenzen zwischen den Positionen 190 bis 200 in der SEQ ID No. 2 durch Aminosäureaustausch verändert sind.

26. DNA gemäß Anspruch 25, **dadurch gekennzeichnet, dass** diese für β-Untereinheiten der RNA-Polymerase B kodieren, wobei die zugehörigen Aminosäuresequenzen an Position 196 in der SEQ ID No. 2 L-Phenylalanin oder L-Tyrosin enthalten.

27. Aus coryneformen Bakterien stammende DNA, kodierend für β-Untereinheiten der RNA-Polymerase B, wobei die zugehörigen Aminosäuresequenzen zwischen den Positionen 420 bis 450 in der SEQ ID No. 2 durch Aminosäureaustausch verändert sind.

28. DNA gemäß Anspruch 27, **dadurch gekennzeichnet, dass** diese für die β-Untereinheiten der RNA-Polymerase B kodieren, wobei die zugehörigen Aminosäuresequenzen an den Positionen 439 in der SEQ ID No. 2 jede andere proteinogene Aminosäure ausgenommen L-Histidin enthalten.

29. DNA gemäß Anspruch 27, **dadurch gekennzeichnet, dass** diese für die β-Untereinheiten der RNA-Polymerase B kodieren, wobei die zugehörigen Aminosäuresequenzen an Position 424 in der SEQ ID No. 2 L-Prolin oder L-Arginin enthalten.

30. DNA gemäß Anspruch 27 **dadurch gekennzeichnet, dass** diese für die β-Untereinheiten der RNA-Polymerase B kodieren, wobei die zugehörigen Aminosäuresequenzen an Position 425 in der SEQ ID No. L-Threonin oder L-Alanin enthalten.

31. DNA gemäß Anspruch 27, **dadurch gekennzeichnet, dass** diese für die β-Untereinheiten der RNA-Polymerase B kodieren, wobei die zugehörigen Aminosäuresequenzen an Position 426 in der SEQ ID No. 2 L-Leucin oder L-Lysin enthalten.

32. DNA gemäß Anspruch 27, **dadurch gekennzeichnet, dass** diese für die β-Untereinheiten der RNA-Polymerase B kodieren, wobei die zugehörigen Aminosäuresequenzen an Position 429 in der SEQ ID No. L-Isoleucin, L-Valin oder L-Leucin enthalten.

33. DNA gemäß Anspruch 27, **dadurch gekennzeichnet, dass** diese für die β-Untereinheiten der RNA-Polymerase B kodieren, wobei die zugehörigen Aminosäuresequenzen an Position 444 in der SEQ ID No. 2 L-Leucin, L- Tyrosin oder L-Tryptophan enthalten.

34. DNA gemäß Anspruch 27, **dadurch gekennzeichnet, dass** diese für die β-Untereinheiten der RNA-Polymerase B kodieren, wobei die zugehörigen Aminosäuresequenzen an Position 446 in der SEQ ID No. 2 L-Prolin oder L-Isoleucin enthalten.

35. Aus coryneformen Bakterien stammende DNA, kodierend für β-Untereinheiten der RNA-Polymerase B, wobei die zugehörigen Aminosäuresequenzen an einer oder mehreren Positionen ausgewählt aus der Gruppe a) Position 1 bis 10, b) Position 190 bis 200 und c) Position 420 bis 450 in der SEQ ID No. 2 gleichzeitig durch Aminosäureaustausch verändert sind.

36. DNA gemäß Anspruch 35, **dadurch gekennzeichnet, dass** diese für die β-Untereinheiten der RNA-Polymerase B kodieren, wobei die zugehörigen Aminosäuresequenzen in der SEQ ID No. 2 an einer oder mehreren Positionen ausgewählt aus der Gruppe a) Position 5 L-Leucin, b) Position 196 L-Phenylalanin, c) Position 429 L-Valin, und d) Position 439 L-Tyrosin, enthalten.

37. DNA gemäß Anspruch 36, **dadurch gekennzeichnet, dass** diese für die β-Untereinheit der RNA-Polymerase B kodiert, wobei die zugehörige Aminosäuresequenz an Position 5 L-Leucin, an Position 196 L-Phenylalanin, und an Position 429 L-Valin, enthält, dargestellt in SEQ ID No. 4.

38. DNA gemäß Anspruch 36, **dadurch gekennzeichnet, dass** diese für die β-Untereinheit der RNA-Polymerase B kodiert, wobei die zugehörigen Aminosäuresequenz an der Position 439 L-Tyrosin enthält, dargestellt in SEQ ID No. 6.

39. Aus coryneformen Bakterien stammende DNA, kodierend für die β-Untereinheit der RNA-Polymerase B, wobei die Basensequenz der DNA an der Position 715 Thymin enthält, an der Position 1288 Thymin enthält, und an der Position 1987 Thymin enthält, dargestellt in SEQ ID No. 3.

40. Aus coryneformen Bakterien stammende DNA, kodierend für die β-Untereinheit der RNA-Polymerase B, wobei die Basensequenz der DNA an der Position 2016 Thymin enthält, dargestellt in SEQ ID No. 5.
